# EUROPEAN PATENT APPLICATION

(11) **EP 2 390 261 A2**
(43) Date of publication of application: **30.11.2011**
(21) Application number: 10733639.8
(22) Date of filing: 20.01.2010
(51) Int. Cl.: C07K 14/505, C12N 15/12, C12N 15/63, A61K 38/17

(54) **MODIFIED HUMAN THROMBOPOIETIN POLYPEPTIDE FRAGMENT AND MANUFACTURING METHOD THEREOF**

(30) Priority: 20.01.2009 KR 20090004812
(71) Applicant: HanAll Biopharma Co., Ltd., Daejeon 306-120 (KR)
(72) Inventor: KIM, Sung Wuk, Seongnam-si Gyeonggi-do 463-802 (KR); JUN, Sung Soo, Seongnam-si Gyeonggi-do 463-777 (KR); PARK, Seung Kook, Seoul 135-942 (KR); JEONG, Jae Kap, Suwon-si Gyeonggi-do 442-833 (KR); LEE, Sung Yul, Suwon-si Gyeonggi-do 441-400 (KR); SONG, Yeon Jung, Suwon-si Gyeonggi-do 443-800 (KR); SHIM, Won Jo, Suwon-si Gyeonggi-do 443-736 (KR)
(74) Representative: von Kreisler Selting Werner
(86) International application number: PCT/KR2010/000368
(87) International publication number: WO 2010/085086

(57) **Abstract**

The present invention provides a modified human thrombopoietin polypeptide fragment that has platelet proliferative activity in vivo and in vitro and enhanced resistance to protease existing in vivo.

## Description

### [Technical Field]

The present invention relates to modified human thrombopoietin polypeptide fragments which are improved in resistance to proteases in vivo and retain the biological activity of promoting the production of platelets in vitro and in vivo, and a method for manufacturing thereof.

More particularly, the present invention relates to modified human thrombopoietin polypeptide fragment consisting of amino acids 1-153 or 7-151, with substitutions at amino acid positions which can confer protease resistance without significant structural alternation, nucleotide sequences encoding the same, and recombinant vectors carrying the genes. Also, the present invention is concerned with a method for manufacturing the modified human thrombopoietin polypeptide fragment variants using the recombinant vectors.

### [Background Art]

Platelets are derived from megakaryocytes in the bone marrow, which is a hemopoietic organ. In the peripheral blood of human beings, normal platelets exist in a range from 150,000 to 450,000 cells/L of peripheral blood. A physiologically constant number of platelets are produced each day, and they have an average lifespan of 7∼10 days. Around one third of the total platelets are stored in the spleen. Functionally, platelets account for hemostasis, leading to the formation of blood clots which fill up a hole in the injured wall of a blood vessel.

Thrombocytopenia is the presence of relatively few platelets in the blood. Generally, thrombocytopenia is defined as a platelet count below 150,000/L. Thrombocytopenia may occur alone, but may occur as an accompaniment to other vascular diseases. Thrombocytopenia may be very rarely congenital, but is, for the most part, postnatal due to secondary causes.

Thrombocytopenia is caused by
i) a decrease in the production of platelets for some reason or other, or
ii) the early destruction of platelets during the circulation thereof in peripheral blood. Normally, three to seven megakaryocytes appear in the field of vision of a low-magnification microscope upon a bone marrow examination. An observation of a lower or a higher number of megakaryocytes serves as an important basis for the opinion of thrombocytopenia due to an increase in the production of platelets or an increase in the destruction of platelets.

Typically, when the platelet count is measured to be over 50,000 cells/L, few significant hemorrhages occur. In contrast, persons with lower than 20,000 platelets/L have to take care of themselves because they may undergo spontaneous hemorrhage even without injury.

Aspects of thromocytopneia due to decreased platelet counts are as follows:

### 1) Medication-induced thrombocytopenia

Medications inducing a decrease in the production of platelets include some diuretics, ethanol, estrogen, sulfa drugs, and anticancer agents while medications inducing the destruction of platelets include quinine, quinidine, heparin, gold, rifampin, sulfa drugs, etc.

### 2) Autoimmune thrombocytopenia

Antibodies against platelets are generated in the plasma of patients and bound to platelets which are then destroyed at an early stage in the spleen. Representative of autoimmne thrombocytopenia is idiopathic thrombocytopenic purpura (ITP). Also, systemic lupus erythematosus, chronic lymphocytic leukemia, and malignant lymphoma may be accompanied by autoimmune thrombocytopenia.

Acute idiopathic thrombocytopenic purpura occurs one to two weeks after viral infection mainly in children. It usually improves spontaneously within six months after the onset thereof even without special treatment. In contrast, chronic idiopathic thrombocytopenic purpura is more common in adults. Unlike acute ITP, chronic ITP develops without special preceding conditions and does not improve spontaneously.

### 3) Thrombocytopenia in pregnancy

Thrombocytopenia is encountered in approximately 5 - 10 % of all pregnancies, but has a significant influence on neither the pregnant woman nor the fetus.

### 4) Acquired Immune Deficiency Syndrome (AIDS)-Related thrombocytopenia

Thrombocytopenia similar to chronic idiopathic thrombocytopenic purpura is frequently generated in patients positive to HIV.

### 5) Blood transfusion-related thrombocytopenia

Thrombocytopenia may occur after multiple transfusions or upon the use of an extracorporeal blood circulator for open heart surgery.

### 6) Other causes

The onset of thrombocytopenia may be induced by other causes including disseminated intravascular coagulation, folic acid or vitamin B12 deficiency, infiltration of tumor cells or tubercle bacillus into the bone marrow, myelofibrosis, malignant blood diseases of bone marrow (leukemia, myelodysplastic syndrome, aplastic anemia, multiple myeloma, paroxysmal nocturnal hemoglobinuria), and other bacterial or viral infection.

Representative among the clinical symptoms of thrombocytopenia is a tendency for hemorrhages such as spontaneous bruising, submucosal hemorrhage, epistaxis, menostaxis, etc.

To date, thrombocytopenia has been treated with steroid agents, or by splenectomy or transfusion. However, such approaches are accompanied by the side effects of steroid agents, or side effects of such as viral infection mediated by transfusion, and immune responses induced by platelet infusions. For the development of a side effect-free regimen of thrombocytopenia, studies on screening the materials directly involved in the production of human platelets, especially on thrombopoietin, have been conducted.

Since the first isolation of the human thrombopoietin gene in the form of cDNA in 1994 (Lok et al., Nature 369:568-571 (1994); de Sauvage et al., Nature 369:533-538 (1994); Bartley et al., Cell 77: 1117-1124 (1994);), active studies have been directed toward megakaryopoiesis and thrombopoiesis (Kuter et al., Proc. Natl. Acad. Sci. USA 91:11104-11108 (1994); Kato et al., J. Biochem. 118:229-236 (1995); Chang et al., J. Biol. Chem. 270:511-514 (1995)).

The human thrombopoietin found in the body has a molecular weight of 60-70 kDa and is a glycoprotein produced mainly by the liver and the kidney that regulates the overall procedure of platelet formation in megakaryocytes differentiated from stem cells. It is expressed in cells as a precursor consisting of 353 amino acid residues which is secreted in a mature form of 332 amino acids, with the cleavage of the 21 amino acid-signal peptide (Bartley et al., Cell 77: 1117-1124 (1994); Chang et al., J. Biol. Chem. 270:511-514 (1995)). Thrombopoietin shows high inter-species sequence homology (Gurney et al., Blood 85:981-988 (1995); Bartley et al., Cell 77:1117-1124 (1994)). Human thrombopoietin shares a sequence homology of 23% with erythropoietin (EPO), a glycoprotein hormone that controls red blood cell production. Human thrombopoietin may be divided into an N-terminal region composed of 153 amino acid residues accounting for the activity of thrombopoietin, and a C-terminal region that plays an important role in the extracellular secretion and in vivo stability of the protein and that has a number of glycosylations (Eaton et al., Exp. Hematol., 25:1-7 (1997); Linden and Kaushansky, J. Biol. Chem., 277: 35240-35247 (2002)).

The following approaches have been tried to improve the activity of human thrombopoietin.

First, a new sugar chain is introduced into human thrombopoietin to enhance the activity of the protein. Typically, many proteins in vivo exist as glycoproteins in which sugar chains are attached to specific residues of the proteins. There are broadly speaking two types of glycosylation: 0-linked glycosylation where a sugar chain is attached to the hydroxyl group of the Ser or Thr residue in the glycoprotein; and N-linked glycosylation where a sugar chain is attached to the amide group of "Asn-X-Ser/Thr" (X is any amino acid except for proline).

The sugar chain in a glycoprotein is known to affect physicochemical properties to play important role in protein stability, activity and secretion (Jenkins et al., Nat. Biotechnol., 14: 975-981 (1996), Dwek, Dev. Biol. Stand., 96:43- 47 (1998)). Amgen INC. and Daewoong Pharmaceutical have attempted to introduce new sugar chains into human thrombopoietin to enhance the activity of human thrombopoietin (International Patent Publication Nos. WO 96/25498 and 00/00612). However, interspecies specificity and environmental characteristics upon overexpression make it difficult to bring about homogeneity in the sugar chains introduced into glycoproteins.

The second method for improving the activity of human thrombopoietin is concerned with the deletion of the C-terminal region or the modification of the N terminus following deletion of the C-terminal. In vitro experiments showed that a human thrombopoietin variant that is devoid of a C-terminal region has higher activity than does the wild-type (Kato et al, Proc. Natl. Acad. Sci. USA, 94:4669-4674 (1997), Muto et al., J. Biol. Chem., 275:12090- 12094 (2000)). In support of this approach, Amgen INC. developed various human thrombopoietin derivatives such as human thrombopoietin 1-151 (consisting of amino acids 1-151 from N-terminus), human thrombopoietin 1-174 (consisting of amino acids 1-174), and human thrombopoietin 1-163 with additional methionine-lysine at its N-terminus. In addition, Genentech, Inc. produced a recombinant human thrombopoietin fragment derivative having an N-terminal methionine by expressing them in E. coli (WO 95/18858). Kirin produced diverse human thrombopoietin derivatives with C-terminal deletion and human thrombopoietin in which there was the substitution of a specific amino acid residue which N-terminal amino acids 1-163 (WO 95/21920). Other human thrombopoietin derivatives with C-terminal deletion were provided by Zymogenetics INC. (WO 95/21920; WO 95/17062) and G. D. Searl (WO 96/23888).

These derivatives, however, are problematic in that the deletion of the C-terminal region abundant in sugar chains gives rise to a decrease in secretion rate upon expression in animal cells and therefore an increase in the opportunity of protease degradation (Linden and Kaushansky, J. Biol. Chem., 277:35240-35247 (2002)).

The third method is associated with the conjugation of human thrombopoietin with polyethyleneglycol (hereinafter referred to as "PEG"). Amgen Inc. reported PEGylated human thrombopoietin 1-163 derivative (WO 95/26746). This method is intended to prevent the weighted derivatives from renal filtration, which is the filtration of materials at a cutoff of 20 kDa by the kidney, thereby prolonging its activity in vivo. However, the qualities of products may be uneven because PEG is not conjugated in uniform proportions.

There are many obstacles for specific protein to be transported to a target with its desired activity intact. Therefore, the delivery of protein agents is a clinically important challenge for pharmacology. During circulation in vivo, protein agents are normally removed by a degradation process including metabolism, glomerular filtration, and degradation by proteases in the gastrointestinal tract, tissues, blood, etc. The enzymatic removal has a great influence on the half life of the protein agents that are administered orally, intravenously, or intramuscularly. In addition, proteases are more apt to attack larger proteins.

Human thrombopoietin, a protein therapeutic, has been developed as an injection for use in promoting the production of platelets. Because injection causes problems, such as pain, infection risk, etc, alternatives, such as lower frequencies of injection, oral administration, etc. are required. In this context, the stability of human thrombopoietin must be increased, but to which degradation by proteases is a great hindrance.

Therefore, one of goals of the development of oral protein agents is to construct a protein with a small number of amino acids resistant to proteases, while maintaining biological activity.

The present inventors succeeded in constructing modified human thrombopoietin polypeptide fragments which show increased resistance to various proteases present in the gastrointestinal tract, cytoplasm and the blood and retain thrombopoietic activity in vivo, which leads to the present invention.

### [Disclosure]

### [Technical Problem]

It is an object of the present invention to provide a modified human thrombopoietin polypeptide fragment which has increased resistance to proteases present in the gastrointestinal tract, cells and blood and retains the biological activity of promoting the production of platelets in vitro and in vivo, a gene encoding the same, a recombinant vector carrying the gene, an animal cell transformed with the recombinant vector, a pharmaceutical formulation comprising the same, a composition for the treatment of thrombocytopenia or for enhancing the production of platelets or megakaryocytes, and a method for treating thrombocytopenia or thrombocytopenia-associated diseases, comprising administering the same.

### [Technical Solution]

### [Definition]

Unless stated otherwise, all technical and scientific terms used in the specification, examples and appended claims have the meanings defined below.

The term "human thrombopoietin" (hereinafter referred to as "TPO"), as used herein, refers to a polypeptide consisting of 332 amino acids, derived from human, which regulates the formation of platelets by binding to cMPL receptors.

The term "human thrombopoietin fragment" or "human thrombopoietin polypeptide fragment" (hereinafter referred to as "TPO fragment" or "TPO polypeptide fragment"), as used herein, refers to a fragment of TPO which has an amino acid sequence 100% homologous to a corresponding amino acid sequence of TPO and which shows a deletion of at least one amino acid residue of the TPO. The deleted amino acid residue(s) may be located at any position of the polypeptide, such as the N-terminus, the C-terminus, or in between these. The fragment shares at least one biological property with full-length TPO. Representative is a fragment composed of a 153-amino acid sequence extending from position 1 to position 153 from N-terminus, called TPO1-153.

As used herein, the term "TPO variant," "TPO fragment variant," "modified TPO,".or "modified TPO fragment" refers to a TPO or a TPO fragment which shares a sequence homology of less than 100% with the TPO or a TPO fragment isolated from the native or recombinant cells as defined below. Typically, the TPO variant has an amino acid sequence with a homology of approximately 70% or higher with TPO or the TPO fragment. The sequence homology is preferably at least approximately 75%, more preferably at least approximately 80%, more preferably at least approximately 85%, even more preferably at least approximately 90%, and most preferably at least approximately 95%.

As used herein, the term "single variant" refers to a TPO variant with a mutation at one position.

As used herein, the term "double variant" refers to a TPO variant with a mutation at two positions.

As used herein, the term "triple variant" refers to a TPO variant with a mutation at three positions.

The term "TPOm-n," as used herein, refers to a TPO fragment having an amino acid sequence extending from position m to position n from N-terminus of the amino acid sequence of TPO. For example, TPO1-153 means a TPO fragment having an amino acid sequence from position 1 to position 153 of the full-length amino acid sequence of TPO. Another example is TP07-151 that has an amino acid sequence extending from position 7 to position 151 of the full-length amino acid sequence of TPO.

The symbol "xAz," as used herein, refers to the substitution of amino acid X at position A with amino acid z. For example, A3S refers to a serine residue (Ser) substituted for an alanine residue (Ala) at position 3.

In accordance with an aspect thereof, the present invention provides a modified TPO polypeptide fragment having increased protease resistance, constructed by substituting one or more amino acid residues of a native TPO fragment which are anticipated to be recognized and cleaved by proteases with one or more amino acid residues which are neither recognized nor cleaved by proteases and which do not significantly change the physicochemical properties.

In the present invention, the use of a TPO polypeptide fragment rather than the full-length TPO as a template for the variants is intended to increase the pharmaceutical bioavailability of TPO. The TPO polypeptide fragment according to the present invention includes macromolecules. Therefore, in order to minimize the likelihood of attack of proteases and to increase its penetration into intestinal epithelial cells and thus to produce injectable preparation and oral preparation, the TPO polypeptide fragment needs to be as small in size as possible. The N-terminal fragment or "EPO homology domain" fragment of TPO is responsible for the biological activity of TPO. The N-terminal fragment has substantially all human TPO sequences between the first and the fourth cysteine residue, but may include significant insertions, deletions or substitutions at the other positions. Hence, it is well known to those of ordinary skill in the art that a region accounting for the pharmaceutical activity of TPO is an amino acid sequence extending from position 7 to position 151. In addition, the C-terminal domain after position 151 is conjugated with many sugar chains. The sugar chains contribute to the stability of the protein, but the inhomogeneous introduction of sugar chains gives rise to increasing pharmaceutical non-uniformity. It is an additional object of the present invention to eliminate such non-uniformity.

The present invention provides a human thrombopoietin (hereinafter referred to as "TPO") fragment variant (TP01-153 variant), having a modified amino acid sequence, wherein the modified amino acid sequence comprises one or more amino acid substitution(s) at position(s) selected from amongst positions 3, 6, 8 to 12, 14 to 18, 20 to 23, 25, 26, 31 to 34, 39 to 41, 43 to 46, 48 to 52, 55 to 57, 59, 60, 62, 64 to 67, 69 to 79, 81, 86, 89 to 91, 93, 95, 97 to 104, 107 to 109, 112, 116, 117, 120 to 123, 126, 128, 129, 131, 133 to 147, 150, and 152 in the amino acid sequence of native TPO fragment 1-153 (hereinafter referred to as "TPO1-153") represented by SEQ ID NO: 1.

In addition, the present invention provides a TPO1-153 fragment variant, having a modified amino acid sequence, wherein the modified amino acid sequence comprises one or more amino acid substitution(s) at position(s) selected from amongst positions 3, 9, 14, 16 to 18, 20 to 23, 25, 32, 40, 43 to 46, 51, 52, 56, 57, 59, 62, 65 to 67, 69, 73 to 76, 79, 81, 99, 103, 104, 109, 117, 122, 129, 133, 135 to 139, and 141 to 144 in the amino acid sequence of TP01-153 represented by SEQ ID NO: 1.

The present invention also provides a TPO1-153 fragment variant, having a modified amino acid sequence, wherein the modified amino acid sequence comprises one or more amino acid substitution(s) at position(s) selected from amongst positions 17, 20, 21, 32, 52, 59, 66, 67, 138, 139, 141, and 142. More preferably, the TPO fragment variant of the present invention comprises one or more amino acid substitution(s) at position(s) selected from amongst positions 21, 52, 138, and 139 in the amino acid sequence of TP1-153 represented by SEQ ID NO: 1.

The present invention also provides a TPO fragment variant (TP07-151 variant) having a modified amino acid sequence, wherein the modified amino acid sequence comprises one or more amino acid substitution(s) at position(s) selected from amongst positions 8 to 12, 14 to 18, 20 to 23, 25, 26, 31 to 34, 39 to 41, 43 to 46, 48 to 52, 55 to 57, 59, 60, 62, 64 to 67, 69 to 79, 81, 86, 89 to 91, 93, 95, 97 to 104, 107 to 109, 112, 116, 117, 120 to 123, 126, 128, 129, 131, 133 to 147, and 150 in the amino acid sequence of TPO1-153 represented by SEQ ID NO: 1 and the amino acid residues at positions 1 to 6, 152 and 153 of the TPO1-153 represented by SEQ ID NO: 1 are deleted.

The present invention also provides a TP07-151 variant having a modified amino acid sequence, wherein the modified amino acid sequence comprises one or more amino acid substitution(s) at position(s) selected from amongst positions 9, 14, 16 to 18, 20 to 23, 25, 32, 33, 40, 43 to 46, 49, 51, 52, 56, 57, 59, 62, 65 to 67, 69, 73 to 76, 78, 79, 81, 97 to 99, 103, 104, 107, 109, 112, 117, 122, 129, 133, and 135 to 145 in the amino acid sequence of TPO1-153 represented by SEQ ID NO: 1 and the amino acid residues at positions 1 to 6, 152 and 153 of the TPO1-153 represented by SEQ ID NO: 1 are deleted.

The present invention also provides a TP07-151 variant having a modified amino acid sequence, wherein the modified amino acid sequence comprises one or more amino acid substitution(s) at position(s) selected from amongst positions 16, 21, 23, 25, 32, 33, 44, 45, 46, 49, 52, 56, 59, 67, 73, 74, 78, 79, 97, 98, 99, 103, 107, 112, 133, 136 to 141, and 145 in the amino acid sequence of TPO1-153 represented by SEQ ID NO: 1 and the amino acid residues at positions 1 to 6, 152 and 153 of the TPO1-153 represented by SEQ ID NO: 1 are deleted. More preferably, the TP07-151 variant of the present invention comprises one or more substitution(s) selected from amongst positions 16, 21, 32, 33, 49, 52, 67, 73, 79, 99, 103, 107, 112, 133, and 145 in the amino acid sequence of TPO1-153 represented by SEQ ID NO: 1.

The present invention also provides a TPO fragment variant having a modified amino acid sequence, wherein the modified amino acid sequence comprises one or more amino acid substitution(s) selected from amongst amino acid substitutions of A with S or T; D with N or Q; L with I; R with Q or N; V with T or I; K with N, Q or T; H with Q or N; E with Q, N, or S; P with S; F with I or S; G with S; W with I or S; and M with I or N at said position(s) in the amino acid sequence of TPO1-153 represented by SEQ ID NO: 1.

Preferably, the above mentioned TPO1-153 variant or TP07-151 variant according to the present invention has a modified amino acid sequence, wherein the modified amino acid sequence comprises one or more amino acid substitution(s) selected from amongst amino acid substitutions of A with S or T; D with N or Q; L with I; R with Q or N; V with T or I; K with N, Q or T; H with Q or N; E with N; P with S; F with I or S; G with S; W with S; and M with I or N; more preferably one or more amino acid substitution(s) selected from amongst amino acid substitutions of A with S; L with I; R with Q; V with T or I; K with N or T; H with Q; and F with I; even more preferably one or more amino acid substitution(s) selected from amongst amino acid substitutions of V with T or I; and K with N or T; most preferably a amino acid substitution of V with T or I in the amino acid sequence of TPO1-153 represented by SEQ ID NO: 1.

The present invention also provides a TPO fragment variant (TPO1-153 variant) having a modified amino acid sequence, wherein the modified amino acid sequence comprises one or more amino acid substitution(s) selected from amongst substitutions of A3S, A3T, L9I, K14N, K14Q, L16I, R17Q, D18Q, H20Q, V21I, V21T, L22I, H23N, R25Q, R25N, V32I, L40I, A43S, V44I, V44T, D45N, F46I, W51S, K52Q, K52N, E56N, E57N, K59N, D62Q, G65S, A66S, V67T, L69I, G73S, V74I, V74T, M75I, A76S, G79S, L81I, L99I, A103T, L104I, G109S, R117Q, K122Q, L129I, H133Q, L135I, R136Q, G137S, K138N, K138Q, K138S, K138T, V139I, V139T, R140Q, F141I, F141S, L142I, M143I, M143N, L144I, V145T, L150I and V152T in the amino acid sequence of TPO1-153 represented by SEQ ID NO: 1.

Preferably, the present invention provides a TPO fragment variant which has a modified amino acid sequence, wherein the modified amino acid sequence comprises one or more amino acid substitution(s) selected from amongst substitutions of R17Q, H20Q, V21T, V32I, K52N, K59N, A66S, V67T, K138Q, K138S, K138T, V139I, V139T, R140Q, F141I, F141S, and L142I; and more preferably one or more amino acid substitution(s) selected from amongst substitutions of V21T, K52N, K138T, and V139I in the amino acid sequence of TPO1-153 represented by SEQ ID NO: 1.

The present invention also provides a TPO1-151 variant having a modified amino acid sequence, wherein the modified amino acid sequence comprises 2 amino acid substitutions in the amino acid sequence of TPO1-153 represented by SEQ ID NO: 1 and the 2 amino acid substitutions are one selected from amongst replacements of V32I/K52N, K52N/K138S or K52N/139I.

The present invention also provides a TPO fragment variant (TP07-151 variant) having a modified amino acid sequence, wherein the modified amino acid sequence comprises one or more amino acid substitution(s) selected from amongst substitutions of L6I, L9I, K14N, K14Q, L16I, R17Q, D18Q, H20Q, V21I, V21T, L22I, H23N, H23Q, R25Q, R25N, V32I, H33N, H33Q, L40I, A43S, V44I, V44T, D45N, F46I, G49S, W51S, K52Q, K52N, E56N, E57N, K59N, K59Q, D62Q, G65S, A66S, V67T, L69I, G73S, V74I, V74T, M75I, A76S, R78Q, G79S, L81I, V97I, R98Q, L99I, A103S, A103T, L104I, L107I, G109S, L112I, R117Q, K122Q, L129I, H133N, H133Q, L135I, R136Q, G137S, K138N, K138Q, K138T, V139I, V139T, R140Q, F141I, F141S, L142I, M143I, M143N, L144I and 145T in the amino acid sequence of TPO1-153 represented by SEQ ID NO: 1 and the amino acid residues at positions 1 to 6, 152 and 153 of the TPO1-153 represented by SEQ ID NO: 1 are deleted.

The present invention also provides a TPO fragment variant (TP07-151 variant) which has a modified amino acid sequence, wherein the modified amino acid sequence comprises one or more amino acid substitution(s) selected from amongst substitutions of L16I, V21I, H23Q, R25Q, V32I, H33N, H33Q, V44T, D45N, F46I, G49S, K52N, E56N, K59N, K59Q, V67T, G73S, V74I, R78Q, G79S, V97I, R98Q, L99I, A103S, A103T, L107I, L112I, H133N, H133Q, R136Q, G137S, K138T, K138Q, V139I, R140Q, F141S, and V145T, and more preferably at least one amino acid substitution selected from amongst L16I, V21I, V32I, H33N, H33Q, G49S, K52N, V67T, G73S, G79S, L99I, A103S, A103T, L107I, L112I, H133Q, and V145T in the amino acid sequence of TPO1-153 represented by SEQ ID NO: 1 and the amino acid residues at positions 1 to 6, 152 and 153 of the TPO1-153 represented by SEQ ID NO: 1 are deleted.

The present invention also provides a TPO fragment variant (TP07-151 variant) which has a modified amino acid sequence, wherein the modified amino acid sequence comprises 2 amino acid substitutions in the amino acid sequence of TPO1-153 represented by SEQ ID NO: 1; the amino acid residues at positions 1 to 6, 152 and 153 of the TPO1-153 represented by SEQ ID NO: 1 are deleted; and the 2 amino acid substitutions are one selected from amongst replacements of V32I/K52N, K52N/K138S or K52N/139I.

The present invention also provides a TPO fragment variant having an amino acid sequence represented by one selected from amongst SEQ ID NOS: 2 to 227.

Further, the present invention provides a gene having a nucleotide sequence encoding the modified amino acid sequence.

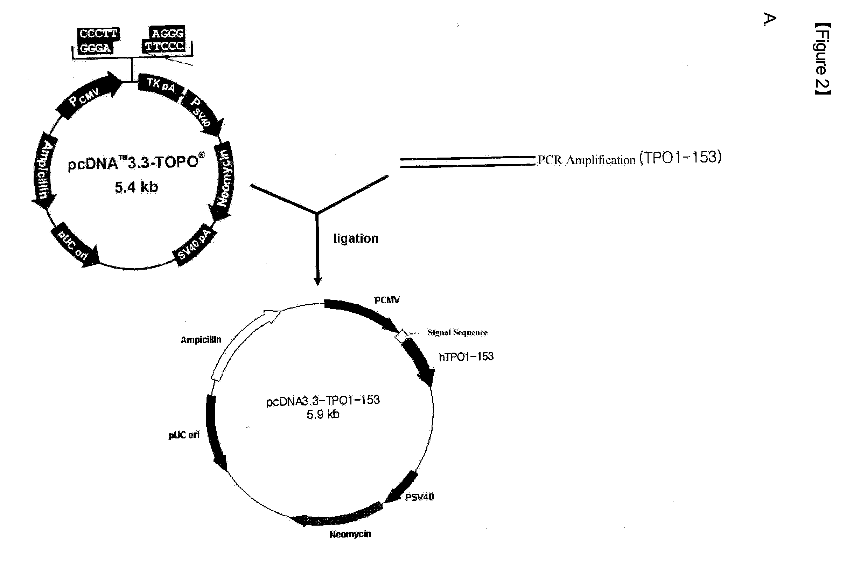

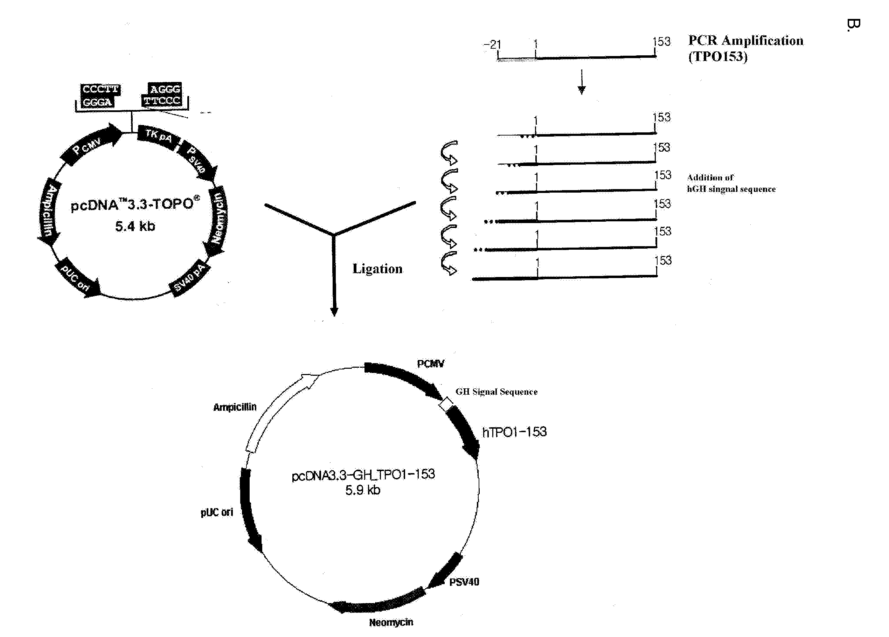

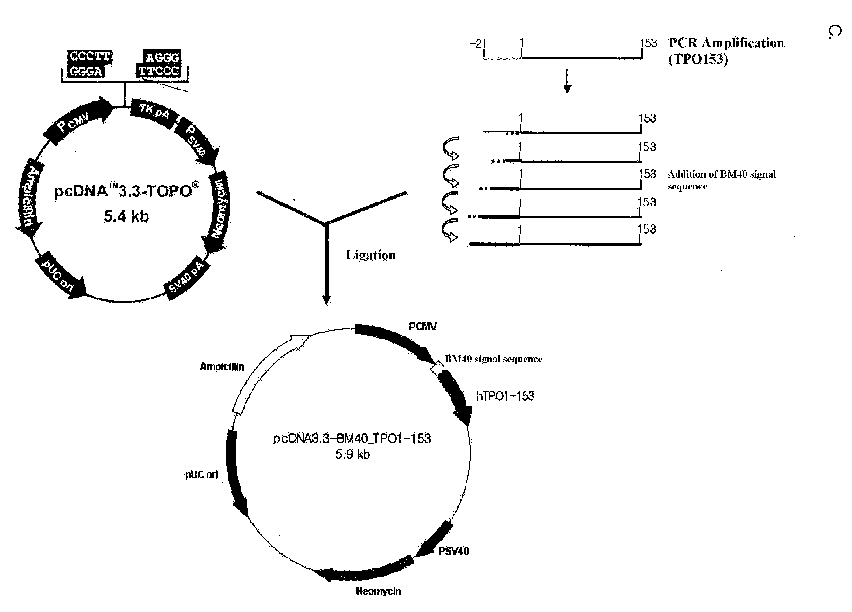

The present invention also provides a vector carrying the gene, preferably a vector, represented by the cleavage map of FIG. 2, carrying a modified TPO gene, and more preferably a vector, represented by the cleavage map of FIG. 2B, carrying a modified TPO gene.

The present invention also provides a microbial or animal cell transformed with the vector, preferably *E.coli* BL21(DE3), a CHO cell, a COS-7 cell and a HEK293 cell, transformed with the vector, and more preferably E. coli BL21(DE3) transformed with the vector (Accession No: KCTC11453BP).

The present invention also provides a method for manufacturing a TPO fragment variant, comprising introducing the gene into a suitable vector, transforming the vector into host cells to give a transformant, and culturing the transformant in a medium to express the TPO fragment variant.

Further, the present invention also provides a pharmaceutical preparation comprising the modified TPO fragment variant as an active ingredient, and preferably further comprising a pharmaceutically acceptable excipient. In the present invention, the pharmaceutical preparation is in the form of a formulation selected from amongst an oral formulation, an inhaler, an injection, a transmucosal formulation, and a external application.

The present invention also provides a composition for the prevention or treatment of thrombocytopenia or thrombocytopenia-associated diseases, comprising a TPO fragment variant having the modified amino acid sequence.

The present invention also provide a method for treating thrombocytopenia or thrombocytopenia-associated diseases, comprising administering a TPO fragment variant having the modified amino acid sequence in a therapeutically effective amount to a patient in need thereof.

In the present invention, the thrombocytopenia or Thrombocytopenia-associated diseases include megakaryocytopenia due to impaired production, sequestration or increased destruction of platelets, megakaryocytopenia-associated bone marrow hypoplasia (e.g. aplastic anemia following chemotherapy or bone marrow transplant), disseminated intravascular coagulation (DIC), immune thrombocytopenia (including HIV-induced ITP and non HIV-induced ITP), chronic idiopathic thrombocytopenia, congenital thrombocytopenia, myelodysplasia and thrombotic thrombocytopenia, thrombocytosis from inflammatory conditions, myeloproliferative thrombocytotic diseases, sideropenia, myelotoxic chemotherapy for treatment of leukemia or solid tumors, myeloablative chemotherapy for autologous or allogeneic bone marrow transplant, myelodysplasia, idiopathic aplastic anemia, congenital thrombocytopenia and immune thrombocytopenia, and defects in or damage to platelets resulting from drugs, poisoning or activation on artificial surfaces.

The TPO fragment variant of the present invention may be employed alone or administered in combination with other cytokines, hematapoietins, interleukins, growth factors, or antibodies in the treatment of the above identified disorders and conditions marked by thrombocytopenia. Thus, the present active materials may be employed in combination with other proteins or peptides having megakaryocytopoietic or thrombopoietic activity including: G-CSF, GM-CSF, LIF, M-CSF, IL-1, IL-3, erythropoietin (EPO), IL-6, IL-11, and so forth.

To produce the TPO fragment variant of the present invention, publicized human TPO gene information was utilized.

The TPO fragment gene of the present invention was amplified by performing a polymerase chain reaction (hereinafter referred to as "PCR") on the cDNA derived from the human fetal male liver gene (Stratagene, Cat. No: 780609-41), which is commercially available. The PCR product thus obtained was cloned into the mammalian expression vector pcDNA 3.3-TOPO TA (Invitrogen, Cat. No: K8300-01), followed by substituting a signal sequence of human growth hormone (hereinafter referred to as "hG") for the TPO signal sequence to construct a recombinant eukaryotic expression vector carrying a TPO gene with improved secretory activity (FIG. 2B). The recombinant vector was transfected into HEK293 cells which were then cultured to express and release the TPO fragment into the medium.

To manufacture a TPO variant resistant to proteases, cleavage sites of the TPO fragment which proteases attack were inferred. In this regard, the amino acid sequence of the TPO fragment was screened for the cleavage sites of 12 representative different proteases located within gastrointestinal tract, cells and blood using the peptide cutter program (http://www.expasy.org/tools/peptidecutter/) provided from Expasy (Expert Protein Analysis System).

To substitute the amino acids at the sites inferred to be cleaved by proteases with amino acids that do not undergo protease cleavage, without significant structural alternations, the amino acids are selected from among the amino acids to which zero or positive numbers are assigned by the PAM250 scoring matrix and that are not recognized by proteases, and the selected amino acids are used as substituents to give the TPO fragment variant of the present invention.

A gene encoding the TPO fragment variant is amplified by PCR and cloned into the mammalian expression vector pcDNA 3.3-TOPO TA (Invitrogen, Cat. No: K8300-01), followed by introducing a signal sequence of hGH, instead of the TPO signal sequence, into the vector to construct an eukaryotic expression vector carrying a TPO gene with improved secretory activity. The recombinant vector is transfected into HEK293 cells which is then cultured to express and release the TPO fragment variant into the culture medium.

Concentrations of the TPO fragment and the TPO fragment variant in the culture media were measured. Predetermined concentrations of the TPO fragment and the TPO fragment variant are applied to the megakaryoblast cell line M-o7e cells to induce STAT5 to be phosphorylated. Measurements of the TPO-mediated phosphorylation of STAT5 exhibit the biological activity of the TPO fragment and its variant (Kamatu et al., Blood 87(11):4552-60 (1996)).

Resistance of the TPO fragment and its variants to proteases was analyzed. The total protein concentration of the medium in which the TPO fragment and its variants are expressed was measured (total proteins in the cell culture medium are quantitatively analyzed using the Bradford method). To the medium, ten different proteases are each added in an amount of 1 % of the total protein concentration. The TPO fragment and its variants are analyzed for half life to determine the protease resistance of the TPO fragment variants compared to the TPO polypeptide fragment.

The present invention also provides methods of using the TPO fragment variants or pharmaceutical compositions comprising them. Such pharmaceutical compositions may be for administration via injection, or for oral, nasal, transdermal or other forms of administration, including, e.g., by intravenous, intradermal, intramuscular, intramammary, intraperitoneal, intrathecal, intraocular, intrapulmonary or subcutaneous injection; by sublingual, anal, vaginal, or by surgical implantation. The treatment may consist of a single dose or a plurality of doses over a period of time. In general, the pharmaceutical compositions of the present invention comprise effective amounts of a compound of the invention together with pharmaceutically acceptable diluents, preservatives, solubilizers, emulsifiers, adjuvants or carriers. Such compositions include diluents of various buffer contents (Tris buffer, acetate buffer, phosphate buffer), detergents (Tween 80), anti-oxidants (ascorbic acid, sodium metabisulfite), preservatives (Thimersal, benzyl alcohol) and bulking substances (lactose, mannitol). In such compositions, the material is incorporated into particulate preparations of polymeric compounds such as polylactic acid, polyglycolic acid, etc. or into liposomes. Hyaluronic acid may also be used, and this may have the effect of promoting sustained duration in the circulation. The pharmaceutical compositions optionally may include still other pharmaceutically acceptable liquid, semisolid, or solid diluents that serve as pharmaceutical vehicles, excipients, or media, including, but not being limited to, polyoxyethylene sorbitan monolaurate, starches, sucrose, dextrose, gum acacia, calcium phosphate, mineral oil, cocoa butter, and oil of theobroma. Such compositions may influence the physical state, stability, rate of in vivo release, and rate of in vivo clearance of proteins and derivatives present in the body. The compositions may be prepared in liquid form, or may be in dried powder, such as lyophilized form. Implantable sustained release formulations are also contemplated, as are transdermal formulations.

Contemplated for use herein are oral solid dosage forms. Solid dosage forms include tablets, capsules, pills, troches or pellets. Also, liposomal or proteinoid encapsulation may be used to formulate the present compositions. Liposomes may be derivatized with various polymers. In general, the formulation includes the composition of the present invention, and inert additives which furnish protection against the stomach environment, and release of the biologically active material in the intestine.

Also specifically contemplated are oral dosage forms of the composition of the present invention. If necessary, the variant may be chemically modified so that oral delivery is efficacious. Generally, the chemical modification contemplated is the attachment of at least one moiety to the variant molecule itself, where the moiety confers resistance to proteolysis, and helps uptake into the blood stream from the stomach or intestine. Also desired is the increase in overall stability of the variant and an increase in its circulation time in the body. Examples of such moieties include polyethylene glycol, copolymers of ethylene glycol and propylene glycol, carboxymethyl cellulose, dextran, polyvinyl alcohol, polyvinyl pyrrolidone and polyproline. Other polymers that could be used are poly-l,3-dioxane and poly-1,3,6-trioxocane. Preferred for pharmaceutical usage, as indicated above, are polyethylene glycol moieties.

For the oral delivery dosage forms, it is also possible to use a salt of a modified aliphatic amino acid, such as sodium N-(8-[2-hydroxybenzoyl] amino) caprylate (SNAC), as a carrier to enhance absorption of the therapeutic variants of this invention. The clinical efficacy of a heparin formulation using SNAC has been demonstrated in a Phase II trial conducted by Emisphere Technologies.

The therapeutics can include in the formulation as fine multiparticulates in the form of granules or pellets of a particle size of about 1 mm. The formulation of the material for capsule administration could also be as a powder, lightly compressed plugs or even as tablets. The therapeutic could be prepared by compression.

Colorants and flavoring agents may also be included. For example, the protein or its derivative may be formulated as a form of, for example, liposome or microsphere encapsulation and then further contained within an edible product, such as a refrigerated beverage containing colorants and flavoring agents.

The volume of the therapeutic may be diluted or increased with an inert material. These diluents could include carbohydrates, especially mannitol, lactose, anhydrous lactose, cellulose, sucrose, modified dextrans and starch. Certain inorganic salts may also be used as fillers including calcium phosphate, magnesium carbonate and sodium chloride. Some commercially available diluents are Fast-Flo, Emdex, Starch1500, Emcompress and Avicell.

Disintegrants may be included in the formulation of the therapeutic into a solid dosage form. Materials used as disintegrants include but are not limited to starch or modified starch such as corn starch, potato starch or pregelatinized starch; clay such as bentonite, montmorillonite, or beegum; celluloses such as microcrystalline cellulose, hydroxypropyl cellulose, or carboxymethyl cellulose; alginates, such as sodium alginate or alginic acid; cross-linked cellulose such as cross carmellose sodium; gums such as guar gum, xanthan gum, etc.; cross-linked polymers such as cross povidone; effervescent agents such as sodium bicarbonate, citric acid, etc., and a combination thereof.

Binders may be used to give the therapeutic agent together as a solid form. Examples of the binders include starch, microcrystalline cellulose, highly dispersable silica, mannitol, sucrose, lactose, polyethylene glycol, polyvinyl pyrrolidone, natural gum, synthetic gum, copovidone, gelatin, hydroxypropylmethyl cellulose (HPMC), hydroxylpropyl cellulose and a mixture thereof.

An antifrictional agent may be included in the formulation of the therapeutic to prevent sticking during the formulation process. Lubricants may include, but are not limited to, stearic acid including its magnesium and calcium salts, polytetrafluoroethylene, liquid paraffin, vegetable oils and waxes. Soluble lubricants may also be used such as sodium lauryl sulfate, magnesium lauryl sulfate, polyethylene glycol of various molecular weights, and Carbowax 4000 and 6000.

Glidants that might improve the flow properties of the drug during its formulation and to aid rearrangement during compression might be added. The glidants may include starch, talc, pyrogenic silicon and hydrated silicoaluminate.

To aid dissolution of the therapeutic into the aqueous environment, a surfactant might be added. Surfactants may include anionic detergents such as sodium lauryl sulfate, dioctyl sodium sulfosuccinate and dioctyl sodium sulfonate. Cationic detergents might be used and could include benzalkonium chloride or benzethonium chloride. The list of potential nonionic detergents that could be included in the formulation as surfactants are as follows: lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methyl cellulose and carboxymethyl cellulose. These surfactants could be present in the formulation of the protein or derivative either alone or as a mixture in different ratios.

Additives which potentially enhance uptake of the composition are for instance fatty acids such as oleic acid, linolenic acid and so on.

A controlled release formulation may be desirable. The drug could be incorporated into an inert matrix which permits release by either diffusion or leaching mechanisms. Slowly disintegrating matrices may also be incorporated into the formulation, e.g., alginates, polysaccharides. Another form of a controlled release of this therapeutic is based on the Oros therapeutic system (Alza Corp.). For example, the drug is enclosed in a semipermeable membrane which allows water to enter and push the drug out through a single small opening due to osmotic effects. Some enteric coatings also have a delayed release effect.

Other coatings may be used for the formulation. These include a variety of sugars. The therapeutic agent could also be given in a film coated tablet and the materials used in this instance are divided into two groups. The first are the nonenteric materials and include methyl cellulose, ethyl cellulose, hydroxyethyl cellulose, methylhydroxy-ethyl cellulose, hydroxypropyl cellulose, hydroxypropyl-methyl cellulose, sodium carboxy-methyl cellulose, providone and polyethylene glycols. The second group consists of the enteric materials that are commonly esters of phthalic acid. In detail, the enteric polymer is selected from the group consisting of an enteric cellulose derivative, an enteric acrylic copolymer, an enteric maleic copolymer, an enteric polyvinyl derivative, and a combination thereof. The enteric cellulose derivative is at least one selected from the group consisting of hypromellose acetate succinate, hypromellose phthalate, hydroxymethylethyl cellulose phthalate, cellulose acetate phthalate, cellulose acetate succinate, cellulose acetate malate, cellulose benzoate phthalate, cellulose propionate phthalate, methylcellulose phthalate, carboxymethylethyl cellulose and ethylhydroxyethyl cellulose phthalate. The enteric acrylic copolymer is at least one selected from the group consisting of a styrene-acrylate copolymer, a methylacrylate-acrylate copolymer, an acrylate methylmethacrylate copolymer, a butyl acrylate-styrene-acrylate terpolymer, a methacrylic acid-methyl methacrylate copolymer (e.g., Eudragit L 100, Eudragit S, Degussa), a methacrylic acid ethyl acrylate copolymer (e.g., Eudragit L 100-55, Degussa), and methyl acrylate-methacrylic acid-octyl acrylate terpolymer. The enteric maleic copolymer is at least one selected from the group consisting of a vinyl acetate-maleic anhydride copolymer, a styrene-maleic anhydride copolymer, a styrene-maleic acid monoester copolymer, a vinylmethylether-maleic anhydride copolymer, an ethylene-maleic anhydride copolymer, a vinylbutylether-maleic anhydride copolymer, an acrylonitrile-methyl crylate maleic anhydride copolymer, and a butyl acrylate-styrene-maleic anhydride terpolymer. The enteric polyvinyl derivative is at least one selected from the group consisting of polyvinylalcohol phthalate, polyvinylacetal phthalate, polyvinylbutyrate phthalate, and polyvinylacetacetal phthalate.

A mixture of materials might be used to provide the optimum film coating. Film coating may be carried out in a pan coater or in a fluidized bed agglomerator or by compression coater.

Also contemplated herein is pulmonary delivery of the present protein or derivatives thereof. The protein or its derivatives are delivered to the lungs of a mammal while inhaling and traverse across the lung epithelial lining to the blood stream.

Contemplated for use in the practice of this invention are a wide range of mechanical devices designed for pulmonary delivery of therapeutic products, including but not limited to, nebulizers, metered dose inhalers, and powder inhalers, all of which are familiar to those skilled in the art.

Some specific examples of commercially available devices suitable for the practice of this invention are the Ultravent nebulizer, manufactured by Mallinckrodt, Inc.; the Acorn II nebulizer, manufactured by Marquest Medical Products; the Ventolin Evohaler, manufactured by Glaxo Inc.; and the Spinhaler powder inhaler, manufactured by Fisons Corp.

All such devices require the use of formulations suitable for the administering of the composition of the present invention. Typically, each formulation is specific to the type of device employed and may involve the use of an appropriate propellant material, in addition to diluents, adjuvants or carriers useful in therapy.

The composition of the present invention should most advantageously be prepared in particulate form with an average particle size of approximately 10 µm or less, most preferably about 0.5 to 5µm, for most effective delivery to the distal lung.

Carriers include carbohydrates such as trehalose, mannitol, xylitol, sucrose, lactose, and sorbitol. Other ingredients for use in formulations may include DPPC, DOPE, DSPC and DOPC. Natural or synthetic surfactants may also be used. Polyethylene glycol may be used even apart from its use in derivatizing the protein or analog. Dextrans, such as cyclodextran, may also be used. Bile salts and other related derivatives may be used. Amino acids may be used, such as are used in a buffer formulation.

Also, the use of liposomes, microcapsules or microspheres, inclusion complexes, or other types of carriers is contemplated.

Formulations suitable for use of a nebulizer, with either jet or ultrasonic, will typically comprise the inventive composition dissolved in water at a concentration of about 0.1 to 25 mg of biologically active protein per mL of solution. The formulation may also include a buffer and monosaccharides, which contributes to protein stabilization and the regulation of osmotic pressure. The nebulizer formulation may also contain a surfactant, to reduce or prevent surface inducing aggregation of the protein caused by atomization of the solution in forming the aerosol.

Formulations for use with a metered-dose inhaler device will generally comprise a finely divided powder containing the composition of the present invention suspended in a propellant with the aid of a surfactant. The propellant may be any conventional material employed for this purpose, such as a chlorofluorocarbon, a hydrochlorofluorocarbon, a hydrofluorocarbon, or a hydrocarbon, including trichlorofluoromethane, dichlorodifluoromethane, dichlorotetrafluoroethanol, and 1,1,1,2-tetrafluoroethane, or combinations thereof. Suitable surfactants include sorbitan trioleate and soya lecithin. Oleic acid may also be useful as a surfactant.

Formulations for dispensing from a powder inhaler device will comprise a finely divided dry powder containing the inventive compound and may also include a bulking agent, such as lactose, sorbitol, sucrose, mannitol, trehalose, or xylitol. These may facilitate dispersal of the powder from the device.

Nasal delivery of the inventive therapeutic is also contemplated. Nasal delivery allows the passage of the protein to the blood stream directly after administering the therapeutic product to the nose, without the necessity for deposition of the product in the lung. Formulations for nasal delivery include dextran or cyclodextran, etc. Delivery via transport across other mucous membranes is also contemplated.

The dosage regimen involved in a method for treating the above-described conditions will be determined by the attending physician, in light of various factors which modify the action of drugs, e.g. the age, condition, body weight, sex and diet of the patient, the severity of any infection, the time of administration and other clinical factors.

The composition of the present invention may be administered by an initial bolus followed by a continuous infusion to maintain therapeutic levels of the drug product in the circulation. As another example, the inventive composition may be administered as a one-time dose. Typical techniques of the art will readily optimize effective dosages and administration regimens as determined by good medical practice and the clinical condition of the individual patient. The frequency of dosing will depend on the pharmacokinetic parameters of the agents and the route of administration. The optimal pharmaceutical formulation will be determined by one skilled in the art depending upon the route of administration and the desired dosage. Such formulations may influence the physical state, stability, rate of in vivo release, and rate of in vivo clearance of the administered agents. For each route of administration, a suitable dose may be calculated according to body weight, body surface area or organ size. Further refinement of the calculations may be necessary to determine the appropriate dosage for treatment. Appropriate dosages may be ascertained thanks to established assays used for determining blood levels dosages in conjunction with appropriate dose-response data. The final dosage regimen will be determined by the attending physician, in light of various factors which modify the action of drugs, e.g. the drug's specific activity, the severity of the damage and the responsiveness of the patient, the age, condition, body weight, sex and diet of the patient and other clinical factors. As studies are conducted, further information will emerge regarding the appropriate dosage levels and duration of treatment for various diseases and conditions.

The therapeutic methods, compositions and variants of the present invention may also be employed, alone or in combination with other cytokines, soluble Mpl receptor, hematopoietic factors, interleukins, growth factors or antibodies in the treatment of disease states characterized by other symptoms as well as thrombocytopenia. It is anticipated that the present invention will prove useful in treating some forms of thrombocytopenia in combination with general stimulators of hematopoiesis, such as IL-3 or GM-CSF.

In cases where the inventive compounds are added to compositions of platelets and/or megakaryocytes and related cells, the amount to be added will generally be ascertained experimentally by techniques and assays known in the art. An exemplary range of amounts is about 0.1 µg - 1 mg of the inventive active ingredient per 10⁶ cells.

The composition of the present invention may be administered via an intravenous or subcutaneous route, in addition to an oral route and by inhalation. Given for systemic administration, the therapeutic composition of the present invention must be free of pyrogens and in a parenterally acceptable solution state with suitable pH, isotonicity and stability. These conditions are well known in the art. In brief, the formulation of the inventive active ingredient is prepared for storage or administration by mixing the desired molecule having an appropriate degree of purity with optional pharmaceutically acceptable carriers, excipients, or stabilizers. Acceptable carriers, excipients, or stabilizers are nontoxic to recipients in the dosages and concentrations employed, and include buffers such as phosphate, citrate, acetate and other organic acid salts; antioxidants such as ascorbic acid; low molecular weight (less than about 10 residues) polypeptides including proteins, such as polyarginine, serum albumin, gelatin, or immunoglobulins; hydrophilic polymers such as polyvinylpyrrolidone; amino acids such as glycine, glutamic acid, aspartic acid, or arginine; and other carbohydrates including monosaccharides, disaccharides, cellulose and derivatives thereof, glucose, mannose, or dextrins; chelating agents such as EDTA; sugar alcohols such as mannitol, or sorbitol; salt-forming counterions such as sodium; and/or non-ionic surfactants such as TWEEN, PLURONICS or polyethylene glycol (PEG).

The megakaryopoietic protein or its mixture in the form of free acid or base or a pharmaceutically acceptable salt is typically formulated at a concentration of about 0.5 to 500 mg/mL in physiologically acceptable vehicles, carriers, excipients, binders, preservatives, stabilizers, flavors, etc. according to conventional pharmaceutical practice. The amount of the active ingredient in the composition is such that it allows for a suitable dose within the indicated range.

Sterile compositions for injection can be formulated according to conventional pharmaceutical practice. For example, solution or suspension of the active compound in a vehicle such as water or naturally occurring vegetable oil like sesame, peanut, or cottonseed oil or a synthetic fatty vehicle like ethyl oleate or the like may be desired. Buffers, preservatives, antioxidants and the like can be incorporated according to accepted pharmaceutical practice.

Suitable examples of sustained-release preparations include semi-permeable matrices of solid hydrophobic polymers containing the protein, which matrices are in the form of shaped articles, e.g., films, or microcapsules. Examples of sustained-release matrices include polyesters, hydrogels [e.g., poly(2-hydroxyethyl-methacrylate) or poly(vinylalcohol) as described by Langer et al., J. Biomed. Mater. Res. 15:167-277, 1981 and Langer, Chem. Tech. 12:98-105, 1982], polylactides (U.S. Patent No. 3,773,919, EP 58,481), copolymers of L-glutamic acid and gamma ethyl-L-glutamate (Sidman et al., Biopolymers 22:547-556, 1983), non-degradable ethylene-vinyl acetate (Langer et al., supra), degradable lactic acid-glycolic acid copolymers (e.g., Lupron Depot^{™} (injectable microspheres composed of lactic acid-glycolic acid copolymer and leuprolide acetate), and poly-D-(-)-3-hydroxybutyric acid (EP 133,988).

While polymers such as ethylene-vinyl acetate and lactic acid-glycolic acid enable release of molecules for over 100 days, some hydrogels release proteins for shorter time periods. When encapsulated proteins remain in the body for a long time, they may denature or aggregate as a result of being exposed to moisture at 37°C, resulting in a loss of biological activity and possible changes in immunogenicity. Rational strategies can be devised for protein stabilization depending on the mechanism involved. For example, if the aggregation mechanism is discovered to form intermolecular S-S bond formation through disulfide interchange, stabilization may be achieved by modifying sulfhydryl residues, lyophilizing from acidic solutions, controlling moisture content, using appropriate additives, and developing specific polymer matrix compositions.

The sustained-released megakaryopoiesis-stimulating protein composition also includes liposomally entrapped megakaryopoietic proteins. Liposomes containing such megakaryopoietic proteins are prepared by methods known per se (DE 3,218,121; Epstein et al, Proc. Natl. Acad. Sci. USA, 82:3688-3692, 1985; Hwang et al, Proc. Natl. Acad. Sci. USA, 77:4030-4034, 1980; EP 52,322; EP 36,676; EP 88,046; EP 143,949; EP 142,641; Japanese patent application No. 83-118008; U.S. Pat. Nos. 4,485,045 and 4,544,545; and EP 102,324). Ordinarily the liposomes are of the small (about 200-800 Å) unilamellar type in which the lipid content is greater than about 30 mol % cholesterol, the selected proportion being adjusted for the sake of the best therapy.

The therapeutically effective dose is determined as above described for the oral dose.

### [Advantageous Effects]

The stable TPO fragment variants of the present invention are modified TPO fragment variants which are constructed by site-directed mutagenesis to have resistance to proteases, have improved bioavailability and adsorption rates upon injection or oral administration because they are less susceptible to protease degradation, and thus can be useful as materials for use in long-acting injection and oral protein medications.

### [Description of Drawings]

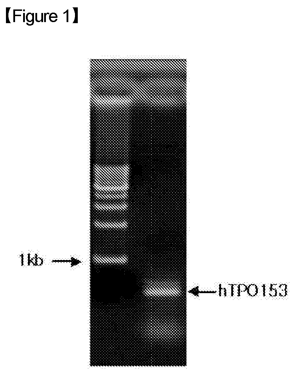

FIG. 1 is a photograph showing 1% agarose on which a TPO153 gene amplified from cDNA of human fetal hepatocytes is run by electrophoresis.
FIG. 2A is a schematic diagram showing a strategy for cloning a TPO1-153 gene into a mammalian expression vector, in which PCR is performed using primer sets described in Example 1, with cDNA isolated from human liver tissues serving as a template, to give the gene which extends from the N-terminal amino acid residue of the signal sequence (s.s) 21 a.a. upstream of the start point of the native TPO gene to the amino acid residue at position 153 of the native TPO protein.
FIG. 2B is a schematic diagram showing a strategy for substituting a hGH signal sequence for the signal sequence which consists of -1 amino acid to -21 amino acid residues and which is located ahead of the TPO153 gene amplified from human liver tissues, in which a gene encoding the hGH signal sequence is sequentially extended in the direction of the 5' end using the primers described in Example 3.
FIG. 2C is a schematic diagram showing a strategy for substituting a BM40 signal sequence for the signal sequence which consists of -1 amino acid to -21 amino acid residues and which is located ahead of the TPO153 gene amplified from human liver tissue, in which a gene encoding the BM40 signal sequence is sequentially extended in the direction of the 5' end using the primers described in Example 3.

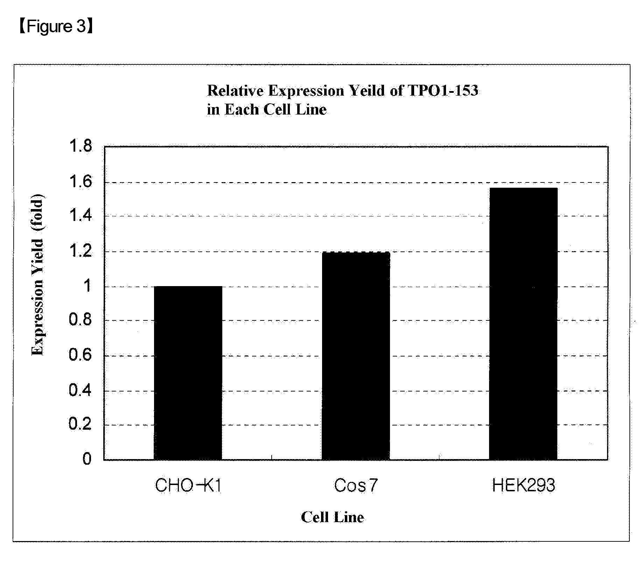

FIG. 3 is a graph showing the expression yields of TPO1-153 in various cell lines transfected with a TPO1-153 expression vector, when analyzing the culture media with ELISA.

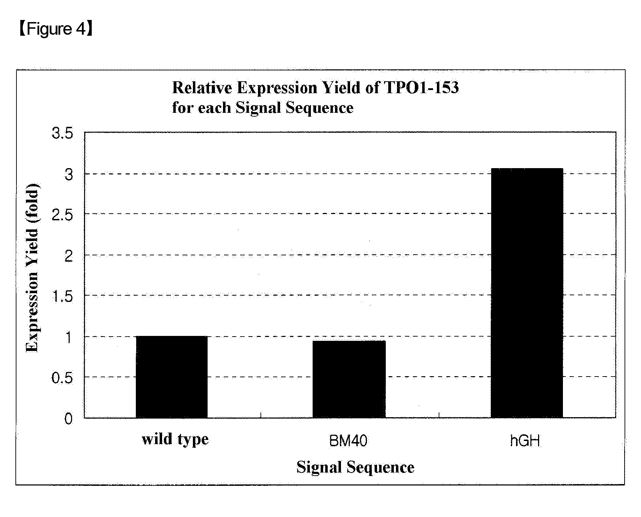

FIG. 4 is a graph showing the expression yields of TPO1-153 in HEK293 cells transfected with expression vectors having the TPO1-153's own signal sequence, the BM40 signal sequence and the hGH signal sequence ahead of the TPO1-153 gene, as the culture media were analyzed by ELISA.

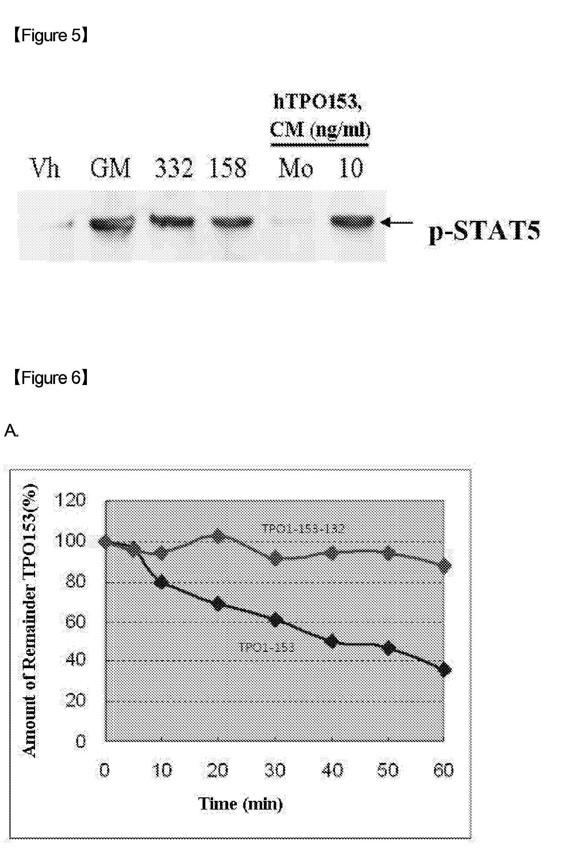

FIG. 5 is a photograph showing the biological activity of TPO1-153 in terms of STAT5 phosphorylation as measured by Western blotting. Vh: Vehicle, RPMI1640; GM: GM-CSF, 10 ng/ml; 332: TPO332, 10 ng/ml (R&D Systems, Cat. No.288-TPN); 158: TPO158 (Antigenix, Cat. No. HC88882B, 10 ng/ml); Mo: Mock transfected CM

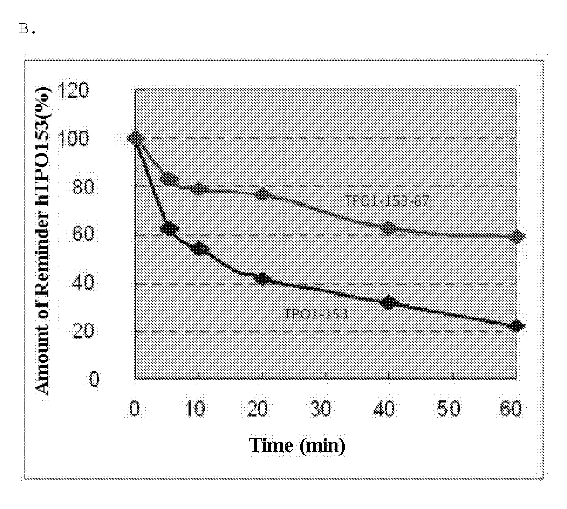

FIG. 6 is of a graph showing the increased protease resistance of mutant TPO1-153, expressed as red lines, mutated at position 132 (A), at position 67 (B) and at position 87
(C), compared to native TPO1-153, represented by blue lines.

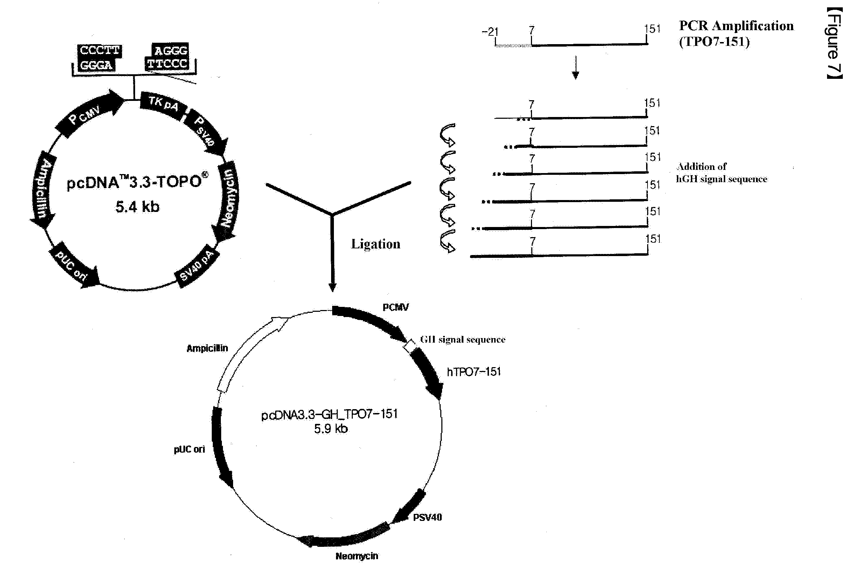

FIG. 7 is a schematic diagram showing a strategy for cloning a TP07-151 gene into a mammalian expression vector, in which PCR is performed using primer sets described in Example 14, with pcDNA3.3-TPO1-153 serving as a template, to give the gene encoding a polypeptide fragment which extends from the amino acid residues at position 7 to position 151 of SEQ ID NO: 1, with a stop codon attached thereto.

### [Best Mode]

The above and other objects, features and other advantages of the present invention will be more clearly understood from the following Example Section. However, the present invention is not limited to the examples disclosed below, but may be embodied in various ways. Although the preferred embodiments of the present invention have been disclosed for illustrative purposes, those skilled in the art will appreciate that various modifications, additions and substitutions are possible, without departing from the scope and spirit of the invention as disclosed in the accompanying claims.

### EXAMPLE 1: Amplification of TPO1-153 Gene

PCR was performed to amplify a gene coding for TPO1-153 where cDNA (Stratagene, Cat#780622), synthesized from the RNA isolated from the human liver, was used as a template (FIG. 1). The primers for use in the PCR amplification were designed to have the nucleotide sequences of accatggagctgactgaattgctcctcgtg (TPO1-153-SN) and ccgctcgagttacctgacgcagagggtggaccctcc (TPO1-153-C), respectively.

More details are given of the PCR, below.

PCR was performed using the primers, with human liver cDNA serving as a template. Each of the primers was dissolved at a concentration of 10 pmol/µL in H₂O. To 3 µL of the primer solution was added Pfu (Pyrococcus furiosus) polymerase (Bioneer, Cat. No. K-2018), together with a PCR premix, to a final volume of 100 µL. The PCR solution had the following composition: 50 ng cDNA, 3 µL 10mM N-primer, 3 µL lOmM C-primer, and 50 µL Accupower PCR premix was dissolved in 39 µL of H₂O to form a final volume of 100 µL.

PCR started with primary denaturing at 95°C for 10 min and was performed with 25 cycles of denaturing at 95°C for 1 min, annealing at 55°C for 1 min and extension at 72°C for 1 min, followed by final extension at 72°C for 5 min.

### [EXAMPLE 2] Construction of TPO1-153 Expression Vector

After completion of the PCR, the PCR mixture was subjected to electrophoresis on 1 % agarose gel, the PCR product of Example 1 was obtained by excising the DNA band at the corresponding size position from the agarose with a razor, and purified with the aid of a DNA isolation kit (GeneAll, Cat. No: 102-102). The PCR product (insert) was mixed at a molar ratio of 3:1 with a pcDNA3.3-TOPO TA vector (Invitrogen, Cat. No: K8300-01), followed by ligation at 14°C for 16 hours in the presence of T4 DNA ligase (Takara, Cat. No: 2011A) to construct a recombinant plasmid which was then transformed into E. coli XL1-blue (RBC, Cat.No: RH119). The recombinant plasmid was isolated from the transformant using a plasmid isolation kit (GeneAll, Cat. No: 101-102) and its sequencing was requested to SolGent (Daejeon Korea) to determine the correct clone. The recombinant plasmid constructed by cloning the TPO1-153 gene into pcDNA3.3-TOPO TA was named pcDNA3.3-TPO1-153 (FIG. 2A). The pcDNA3.3-TPO1-153 was used as a template for use in the addition of signal sequences of hGH and BM40 thereto or for constructing site-specific mutants using the primers listed in Table 3.

### [EXAMPLE 3] Construction of TPO1-153 Expression Vector Having Signal Sequence of Human Growth Hormone

To improve the secretion of the TPO fragment, a signal sequence of hGH was introduced into the N terminus of the TPO gene cloned in pcDNA3.3-TPO1-153 using PCR, in substitution for TPO's own signal sequence.

Primers for use in this PCR were TPO1-153hGH-N1 (ttcaagagggcagtgccagcccggctcctcctgcttgt), TPO1-153hGH-N2 (tctgcctgccctggcttcaagagggcagtgccagcc), TPO1-153hGH-N3 (tggcttttggcctgctctgcctgccctggcttcaag), TPO1-153hGH-N4 (ggacgtccctgctcctggcttttggcctgctctgcc), TPO1-153hGH-N5 (accatggctacaggctcccggacgtccctgctcctggct) and TPO1-153-C (ccgctcgagttacctgacgcagagggtggaccctcc). PCR was performed as described for the construction of pcDNA3.3-TPO1-153. In brief, a gene of interest was amplified by PCR using a pair of primers TPO1-153hGH-Nl and TPO1-153-C and the PCR product was purified with the aid of Expin PCR SV (GeneAll, Cat. No: 103-102). Secondary PCR was performed on the purified primary PCR product with a pair of primers TPO1-153hGH-N2 and TPO1-153-C. Like this, PCR with a serial of primers from 153hGH-N1 to 153hGH-N5 gave a final PCR product having a signal sequence of hGH that was then cloned into a pcDNA3.3 TOPO TA vector to construct a recombinant vector called pcDNA3.3-GH_TPO1-153 (FIG. 2B). Base sequencing confirmed the cloning of a correct hGH signal sequence.

### [EXAMPLE 4] Construction of TPO1-153 Expression Vector Having Signal Sequence of BM40

To improve the secretion of the TPO fragment, a signal sequence of BM40 was introduced into the N terminus of the TPO gene cloned in pcDNA3.3-TPO1-153 using PCR, in substitution for TPO's own signal sequence.

Primers for use in this PCR were TPO fragment BM40-Nl (gccttggcagcccctagcccggctcctcctgcttgtg), TPO fragment BM40-N2 (tgcctggccgggagggccttggcagcccctagccc), TPO fragment BM40-N3 (atcttctttctcctttgcctggccgggagggcctt), TPO fragment BM-N4 (accatgagggcctggatcttctttctcctttgcct), and TPO fragment-C (ccgctcgagttacctgacgcagagggtggaccctcc). After the complete signal sequence of BM40 was introduced by PCR using a serial of the primers, the final PCR product was inserted into pcDNA3.3-TOPO TA as described for the insertion of the hGH signal sequence in Example 3 to construct a recombinant vector called pcDNA3.3-BM40_TPO1-153 (FIG. 2C). Base sequencing confirmed the cloning of a correct BM40 signal sequence.

### [EXAMPLE 5] Selection of TPO1-153 Expression Animal Cell Line

Animal cell lines having good secretion rate should be selected to increase expression and secretion rates for TPO1-153. In this regard, pcDNA3.3-TPO1-153 vector was transfected transiently into the mammalian cell lines CHO-K1, COS7 and HEK293 (all from the Korean Cell Line Bank (KCLB), Korea) to examine the expression level of the gene of interest.

In detail, after being isolated, the pcDNA3.3-TPO1-153 plasmid constructed in Example 2 was dissolved at a concentration of 500 ng/µL. COS7 and HEK293 cells were maintained in a DMEM medium (Gibco, Cat. No.: 11995-065) supplemented with 10% FBS (Gibco Cat. No. 26140-079) while CHO-K1 cells were maintained in RPMI1640 (Gibco, Cat. No: 42401). One day prior to transfection, the cells were seeded at a density of 200,000 cells/well on 6-well plates. Next day, 4 µg of DNA and 2.5 µL of Enhancer-Q (Welgene, Cat. No. TR-003) were added to 50 µL of OPTI-MEM (Invitrogen, Cat. No. 31985) per well and incubated at room temperature for 15 min. To each well was added 2 µL of WelFect-Ex (Welgene, Cat. No. TR-003-01), followed by incubation at room temperature for 10 min. In the meantime, the media for HEK293, CHO-K1, and COS7 cells were removed, and 1 mL of OPTI-MEM was added to each well. A mixture of DNA and liposomes was introduced into cells and incubated for 4 hours, after which 10% FBS supplemented DMEM and RPMI1640 media were replaced with fresh ones. The cells were incubated for 72 hours, and the supernatant was harvested and quantitatively analyzed for the expression level of the protein in the same manner as described in Example 10. As a result, HEK293 was selected to use for the expression of TPO1-153 because it had the highest expression rate (FIG. 3).

### [EXAMPLE 6] Comparison of Secretion Rate According to Signal Sequences Employed

The recombinant vectors having the signal sequences of TPO, hGH and BM40, respectively constructed in Examples 2, 3 and 4, were transiently transfected into HEK293 in the same manner as in Example 5 to examine the expression rates of TPO1-153.

In brief, one day before transfection, HEK293 cells cultured in 10% FBS-supplemented DMEM medium were seeded at a density of 200,000 cells/well on 6-well plates. Next day, 4 µg of each plasmid DNA and 2.5 µL of Enhancer-Q were added to 50 µL of OPTI-MEM per well and incubated at room temperature for 15 min. To each well was added 2 µL of WelFect-Ex, followed by incubation at room temperature for 10 min. In the meantime, the medium for HEK293 were removed, and 1 mL of OPTI-MEM was added to each well. A mixture of DNA and liposomes was introduced into cells and incubated for 4 hours, after which 10% FBS supplemented DMEM was replaced with a fresh one. The cells were incubated for 72 hours, and the supernatant was harvested and quantitatively analyzed for the expression level of TPO1-153.

HEK293 cells, when transfected with pcDNA3.3-GH_TPO1-153, were found to express and secrete the protein at a rate 3-fold higher than when transfected with native pcDNA3.3-TPO1-153 or pcDNA3.3-BM40_TPO1-153 (FIG. 4).

### [EXAMPLE 7] Design of TPO1-153 Variants

Variants resistant to proteases were first designed. To determine mutation sites, first, the amino acid sequence of TPO1-153 was screened for the cleavage sites that are attacked by 12 representative different proteases located within the gastrointestinal tract, cells and the blood, using the peptide cutter program (http://www.expasy.org/tools/peptidecutter/) provided from Expasy (Expert Protein Analysis System). Then, the amino acids of the sites inferred to be cleaved by proteases were replaced by ones that do not undergo protease cleavage, without significant structural alternations. The substitution amino acids were selected using the PAM250 scoring matrix (W. A Pearson, Rapid and Sensitive Sequence Comparison with FASTP and FASTA, in Methods in Enzymology, ed. R. Doolittle (ISBN 0-12-182084-X, Academic Press, San Diego) 183: 63-98 (1990)), in which 19 amino acid residues are assigned negative, zero and positive values for each of 20 amino acid residues. The amino acids to which zero or positive numbers are assigned by the PAM250 scoring matrix and that are not recognized by proteases were selected as substituents to give the TPO fragment variants of the present invention.

**TABLE 1 Protease-Recognizable and Resistant Amino Acids**

| Protease | Recognizable amino acid | Resistance-inducible substitution amino acid |
|---|---|---|
| Arg-C proteinase | R | N or Q |
| Asp-N endopeptidase | -D | N or Q |
| Chymotrypsin | [FYWML], not befor P | I |
| | [H], not before P | N or Q |
| Enderokinase | K | N or Q |
| Factor Xa | R | N or Q |
| Glutamyl endopeptidase | E | Q |
| LysC | K | N or Q |
| LysN | K | N or Q |
| Proline-endopeptidase | H, K or R-P | S or T |
| Thrombin | R | N or Q |
| Trypsin | K | N or Q |
| Elastase | A | S or T |
| | G | S |
| | V | I or T |

The TPO1-153 variants designed according to the above-mentioned method are listed in Table 2, below.

**TABLE 2 Designed TPO1-153 Single variants**

| Variant No. | Mutation | Variant No. | Mutation |
|---|---|---|---|
| TPO1-153-1 | A3S | TPO1-153-75 | E72N |
| TPO1-153-2 | A3T | TPO1-153-76 | E72S |
| TPO1-153-3 | A6S | TPO1-153-77 | G73S |
| TPO1-153-4 | A6T | TPO1-153-78 | V74I |
| TPO1-153-5 | D8Q | TPO1-153-79 | V74T |
| TPO1-153-6 | D8N | TPO1-153-80 | M75I |
| TPO1-153-7 | L9I | TPO1-153-81 | A76S |
| TPO1-153-8 | R10Q | TPO1-153-82 | A76T |
| TPO1-153-9 | V11I | TPO1-153-83 | A77S |
| TPO1-153-10 | V11T | TPO1-153-84 | A77T |
| TPO1-153-11 | L12I | TPO1-153-85 | R78Q |
| TPO1-153-12 | K14N | TPO1-153-86 | G79S |
| TPO1-153-13 | K14Q | TPO1-153-87 | L81I |
| TPO1-153-14 | L15I | TPO1-153-88 | L86I |
| TPO1-153-15 | L16I | TPO1-153-89 | L89I |
| TPO1-153-16 | R17Q | TPO1-153-90 | L90I |
| TPO1-153-17 | D18N | TPO1-153-91 | G91S |
| TPO1-153-18 | D18Q | TPO1-153-92 | L93I |
| TPO1-153-19 | H20N | TPO1-153-93 | G95S |
| TPO1-153-20 | H20Q | TPO1-153-94 | V97I |
| TPO1-153-21 | V21I | TPO1-153-95 | V97T |
| TPO1-153-22 | V21T | TPO1-153-96 | R98Q |
| TPO1-153-23 | L22I | TPO1-153-97 | L99I |
| TPO1-153-24 | H23N | TPO1-153-98 | L100I |
| TPO1-153-25 | H23Q | TPO1-153-99 | L101I |
| TPO1-153-26 | R25Q | TPO1-153-100 | G102S |
| TPO1-153-27 | R25N | TPO1-153-101 | A103S |
| TPO1-153-28 | L26I | TPO1-153-102 | A103T |
| TPO1-153-29 | E31Q | TPO1-153-103 | L104I |
| TPO1-153-30 | V32I | TPO1-153-104 | L107I |
| TPO1-153-31 | V32T | TPO1-153-105 | L108I |
| TPO1-153-32 | H33N | TPO1-153-106 | G109S |
| TPO1-153-33 | H33Q | TPO1-153-107 | L112I |
| TPO1-153-34 | P34S | TPO1-153-108 | G116S |
| TPO1-153-35 | V39I | TPO1-153-109 | R117Q |
| TPO1-153-36 | V39T | TPO1-153-110 | A120S |
| TPO1-153-37 | L40I | TPO1-153-111 | A120T |
| TPO1-153-38 | L41I | TPO1-153-112 | H121N |
| TPO1-153-39 | A43S | TPO1-153-113 | H121Q |
| TPO1-153-40 | A43T | TPO1-153-114 | K122N |
| TPO1-153-41 | V44I | TPO1-153-115 | K122Q |
| TPO1-153-42 | V44T | TPO1-153-116 | D123Q |
| TPO1-153-43 | D45Q | TPO1-153-117 | D123N |
| TPO1-153-44 | D45N | TPO1-153-118 | A126S |
| TPO1-153-45 | F46I | TPO1-153-119 | A126T |
| TPO1-153-46 | L48I | TPO1-153-120 | F128I |
| TPO1-153-47 | G49S | TPO1-153-121 | L129I |
| TPO1-153-48 | E50Q | TPO1-153-122 | L129V |
| TPO1-153-49 | E50N | TPO1-153-123 | F131I |
| TPO1-153-50 | W51I | TPO1-153-124 | H133N |
| TPO1-153-51 | W51S | TPO1-153-125 | H133Q |
| TPO1-153-52 | K52Q | TPO1-153-126 | L134I |
| TPO1-153-53 | K52N | TPO1-153-127 | L135I |
| TPO1-153-54 | M55I | TPO1-153-128 | R136Q |
| TPO1-153-55 | E56Q | TPO1-153-129 | G137S |
| TPO1-153-56 | E56N | TPO1-153-130 | K138N |
| TPO1-153-57 | E57Q | TPO1-153-131 | K138Q |
| TPO1-153-58 | E57N | TPO1-153-132 | K138T |
| TPO1-153-59 | K59Q | TPO1-153-133 | V139I |
| TPO1-153-60 | K59N | TPO1-153-134 | V139T |
| TPO1-153-61 | A60S | TPO1-153-135 | R140Q |
| TPO1-153-62 | A60T | TPO1-153-136 | F141I |
| TPO1-153-63 | D62Q | TPO1-153-137 | F141S |
| TPO1-153-64 | D62N | TPO1-153-138 | L142I |
| TPO1-153-65 | L64I | TPO1-153-139 | M143I |
| TPO1-153-66 | G65S | TPO1-153-140 | M143N |
| TPO1-153-67 | A66S | TPO1-153-141 | L144I |
| TPO1-153-68 | A66T | TPO1-153-142 | V145I |
| TPO1-153-69 | V67I | TPO1-153-143 | V145T |
| TPO1-153-70 | V67T | TPO1-153-144 | G146S |
| TPO1-153-71 | L69I | TPO1-153-145 | G147S |
| TPO1-153-72 | L70I | TPO1-153-146 | L150I |
| TPO1-153-73 | L71I | TPO1-153-147 | V152I |
| TPO1-153-74 | E72Q | TPO1-153-148 | V152T |
| | | TPO1-153-149 | K138S |

### [EXAMPLE 8] Construction of TPO1-153 Single variants

Site-specific TPO1-153 Single variants were constructed by site-directed mutagenesis using the primers of Table 3 which were designed to correspond to the mutation of each variant.

In more detail, site-specific variants were constructed by PCR using the primers, with pcDNA3.3-GH_TPO1-153 serving as a template. Each of the primers was dissolved at a concentration of 10 pmol/µL in H₂O. To 3 µL of the primer solution was added Pfu polymerase, together with a PCR premix, to a final volume of 50 µL. The PCR solution had the following composition: 1 µL of 50 ng pcDNA3.3-GH_TPO1-153 DNA, 0.25 µL of 10 pmole N-primer, 0.25 µL of 10 pmole C-primer, and 25 µL of 2X PrimeSTAR PCR premix, 2 µL of 200 µM dNTP, and 0.5 µL of PrimeSTAR HS DNA polymerase (Takara, Cat. No: R044A) was dissolved in 19 µL of H₂O to form a final volume of 50 µL.

PCR started with primary denaturing at 99°C for 10 sec and was performed with 22 cycles of denaturing at 98°C for 10 sec, annealing at 68°C for 30 sec and extension at 74°C for 5.5 min, followed by final extension at 74°C for 7 min.

Thereafter, the PCR solutions were treated for 5 min with DpnI to degrade the DNA of E. coli. The PCR products thus obtained were introduced into E. coli XL1-blue cells. The recombinant plasmids isolated from the transformants were subjected to base sequencing to confirm the site-directed mutagenesis.

**TABLE 3 Primers for Site-Directed Mutagenesis**

| Primer No. | Mutation | Direction | Sequence |
|---|---|---|---|
| 1 | A3S | Forward | 5'-gcagtgccagcccgAGCcctcctgcttgtg-3' |
| | | Reverse | 5'-cacaagcaggaggGCTcgggctggcactgc-3 , |
| 2 | A3T | Forward | 5'-gcagtgccagcccgACCcctcctgcttgtg-3' |
| | | Reverse | 5'-cacaagcaggaggGGTcgggctggcactgc-3' |
| 3 | A6S | Forward | 5'-gcccggctcctcctAGCtgtgacctccgag-3' |
| | | Reverse | 5'-ctcggaggtcacaGCTaggaggagccgggc-3' |
| 4 | A6T | Forward | 5'-cccggctcctcctACCtgtgacctccg-3' |
| | | Reverse | 5'-cggaggtcacaGGTaggaggagccggg-3' |
| 5 | D8Q | Forward | 5'-ctcctcctgcttgtCAGctccgagtcctcag-3' |
| | | Reverse | 5'-ctgaggactcggagCTGacaagcaggaggag-3' |
| 6 | D8N | Forward | 5'-cggctcctcctgcttgtAATctccgag-3' |
| | | Reverse | 5'-ctcggagATTacaagcaggaggagccg-3' |
| 7 | L9I | Forward | 5'-cctcctgcttgtgacATTcgagtcctcagta-3' |
| | | Reverse | 5'-tactgaggactcgAATgtcacaagcaggagg-3' |
| 8 | R10Q | Forward | 5'-cctgcttgtgacctcCAGgtcctcagtaaactg-3' |
| | | Reverse | 5'-cagtttactgaggacCTGgaggtcacaagcagg-3' |
| 9 | V11I | Forward | 5'-tcctgcttgtgacctccgaATTctcagtaaactg-3' |
| | | Reverse | 5'-cagtttactgagAATtcggaggtcacaagcagga-3' |
| 10 | V11T | Forward | 5'-ctgcttgtgacctccgaACCctcagtaaactgct-3' |
| | | Reverse | 5'-agcagtttactgagGGTtcggaggtcacaagcag-3' |
| 11 | L12I | Forward | 5'-cttgtgacctccgagtcATCagtaaactgcttcg-3' |
| | | Reverse | 5'-cgaagcagtttactGATgactcggaggtcacaag-3' |
| 12 | K14N | Forward | 5'-ctccgagtcctcagtAATctgcttcgtgactcc-3' |
| | | Reverse | 5'-ggagtcacgaagcagATTactgaggactcggag-3' |
| 13 | K14Q | Forward | 5'-ctccgagtcctcagtCAGctgcttcgtgactccc-3' |
| | | Reverse | 5'-gggagtcacgaagcagCTGactgaggactcggag-3' |
| 14 | L15I | Forward | 5'-cgagtcctcagtaaaATCcttcgtgactcccatg-3' |
| | | Reverse | 5'-catgggagtcacgaagGATtttactgaggactcg-3' |
| 15 | L16I | Forward | 5'-agtcctcagtaaactgATCcgtgactcccatgtc-3' |
| | | Reverse | 5'-gacatgggagtcacgGATcagtttactgaggact-3' |
| 16 | R17Q | Forward | 5'-ctcagtaaactgcttCAGgactcccatgtccttc-3' |
| | | Reverse | 5'-gaaggacatgggagtcCTGaagcagtttactgag-3' |
| 17 | D18N | Forward | 5'-gtcctcagtaaactgcttcgtAATtcccatgtcc-3' |
| | | Reverse | 5'-ggacatgggaATTacgaagcagtttactgaggac-3' |
| 18 | D18Q | Forward | 5'-ctcagtaaactgcttcgtCAGtcccatgtccttc-3' |
| | | Reverse | 5'-gaaggacatgggaCTGacgaagcagtttactgag-3' |
| 19 | H20N | Forward | 5'-actgcttcgtgactccAATgtccttcacagcag-3' |
| | | Reverse | 5'-ctgctgtgaaggacATTggagtcacgaagcagt-3' |
| 20 | H20Q | Forward | 5'-gcttcgtgactccCAGgtccttcacagcaga-3' |
| | | Reverse | 5'-tctgctgtgaaggacCTGggagtcacgaagc-3' |
| 21 | V21I | Forward | 5'-ctgcttcgtgactcccatATCcttcacagcaga-3' |
| | | Reverse | 5'-tctgctgtgaagGATatgggagtcacgaagcag-3' |
| 22 | V21T | Forward | 5'-tgcttcgtgactcccatACCcttcacagcagac-3' |
| | | Reverse | 5'-gtctgctgtgaagGGTatgggagtcacgaagca-3' |
| 23 | L22I | Forward | 5'-tcqtqactcccatqtcATTcacaqcaqactqaq-3' |
| | | Reverse | 5'-ctcagtctgctgtgAATgacatgggagtcacga-3' |
| 24 | H23N | Forward | 5'-cttcgtgactcccatgtccttAATagcagactga-3' |
| | | Reverse | 5'-tcagtctgctATTaaggacatgggagtcacgaag-3' |
| 25 | H23Q | Forward | 5'-cgtgactcccatgtccttCAGagcagactgag-3' |
| | | Reverse | 5'-ctcagtctgctCTGaaggacatgggagtcacg-3' |
| 26 | R25Q | Forward | 5'-catgtccttcacagcCAGctgagccagtgcccag-3' |
| | | Reverse | 5'-ctgggcactggctcagCTGgctgtgaaggacatg-3' |
| 27 | R25N | Forward | 5'-catgtccttcacagcAATctgagccagtgcccag-3' |
| | | Reverse | 5'-ctgggcactggctcagATTgctgtgaaggacatg-3' |
| 28 | L26I | Forward | 5'-cccatgtccttcacagcagaATCagccagtgccc-3' |
| | | Reverse | 5'-gggcactggctGATtctgctgtgaaggacatggg-3' |
| 29 | E31Q | Forward | 5'-ctgagccagtgcccaCAGgttcaccct-3' |
| | | Reverse | 5'-agggtgaacCTGtgggcactggctcag-3' |
| 30 | V32I | Forward | 5'-qccaqtqcccaqaqATTcaccctttqcct-3' |
| | | Reverse | 5'-aggcaaagggtgAATctctgggcactggc-3' |
| 31 | V32T | Forward | 5'-gagccagtgcccagagACCcaccctttgcctaca-3' |
| | | Reverse | 5'-tgtaggcaaagggtgGGTctctgggcactggctc-3' |
| 32 | H33N | Forward | 5'-aqccaqtqcccaqaqqttAATcctttqcc-3' |
| | | Reverse | 5'-ggcaaaggATTaacctctgggcactggct-3' |
| 33 | H33Q | Forward | 5'-ccagtgcccagaggttCAGcctttgccta-3' |
| | | Reverse | 5'-taggcaaaggCTGaacctctgggcactgg-3' |
| 34 | P34S | Forward | 5'-gtgcccagaggttcacAGCttgcctacacctgtc-3' |
| | | Reverse | 5'-gacaggtgtaggcaaGCTgtgaacctctgggcac-3' |
| 35 | V39I | Forward | 5'-accctttgcctacacctATCctgctgcctg-3' |
| | | Reverse | 5'-caggcagcagGATaggtgtaggcaaagggt-3' |
| 36 | V39T | Forward | 5'-ccctttgcctacacctACCctgctgcctgctg-3' |
| | | Reverse | 5'-cdgcdggcdgcdgGGTdggtgtdggcdddggg-3' |
| 37 | L40I | Forward | 5'-tgcctacacctgtcATCctgcctgctgtggactt-3' |
| | | Reverse | 5'-aagtccacagcaggcagGATgacaggtgtaggca-3' |
| 38 | L41I | Forward | 5'-cctacacctgtcctgATCcctgctgtggacttta-3' |
| | | Reverse | 5'-taaagtccacagcaggGATcaggacaggtgtagg-3' |
| 39 | A43S | Forward | 5'-cacctgtcctgctgcctAGCgtggactttagctt-3' |
| | | Reverse | 5'-aagctaaagtccacGCTaggcagcaggacaggtg-3' |
| 40 | A43T | Forward | 5'-cctgtcctgctgcctACCgtggactttagct-3' |
| | | Reverse | 5'-agctaaagtccacGGTdggcdgcdggdcdgg-3' |
| 41 | V44I | Forward | 5'-gtcctgctgcctgctATCgactttagcttggga-3' |
| | | Reverse | 5'-tcccaagctaaagtcGATdgcdggcdgcdggdc-3' |
| 42 | V44T | Forward | 5'-ctgtcctgctgcctgctACCgactttagcttggg-3' |
| | | Reverse | 5'-cccaagctaaagtcGGTagcaggcagcaggacag-3' |
| 43 | D45Q | Forward | 5'-ctgctgcctgctgtgCAGtttagcttgggagaat-3' |
| | | Reverse | 5'-attctcccaagctaaaCTGcacagcaggcagcag-3' |
| 44 | D45N | Forward | 5'-ctgctgcctgctgtgAATtttagcttgggag-3' |
| | | Reverse | 5'-ctcccaagctaaaATTcdcdgcdggcdgcdg-3' |
| 45 | F46I | Forward | 5'-gctgcctgctgtggdcATTdgcttgggdgddtg-3' |
| | | Reverse | 5'-cattctcccaaqctAATqtccacaqcaqqcaqc-3' |
| 46 | L48I | Forward | 5'-qtqqactttaqcATTqqaqaatqqaaaacccaq-3' |
| | | Reverse | 5'-ctgggttttccattctccAATgctaaagtccac-3' |
| 47 | G49S | Forward | 5'-ctgtggactttagcttgAGCgaatggaaaaccca-3' |
| | | Reverse | 5'-tgggttttccattcGCTcaagctaaagtccacag-3' |
| 48 | E50Q | Forward | 5'-gtggactttagcttgggaCAGtggaaaacccaga-3' |
| | | Reverse | 5'-tctgggttttccaCTGtcccaagctaaagtccac-3' |
| 49 | E50N | Forward | 5'-ggactttagcttgggaAATtggaaaacccagatg-3' |
| | | Reverse | 5'-catctgggttttccaATTtcccaagctaaagtcc-3' |
| 50 | W51I | Forward | 5'-ctttagcttgggagaaATTaaaacccagatggag-3' |
| | | Reverse | 5'-ctccatctgggttttAATttctcccaagctaaag-3' |
| 51 | W51S | Forward | 5'-ctttagcttgggagaaAGCaaaacccagatggag-3' |
| | | Reverse | 5'-ctccatctgggttttGCTttctcccaagctaaag-3' |
| 52 | K52Q | Forward | |
| | | Reverse | 5'-ggtctcctccatctgggtCTGccattctcccaagc- 3' |
| 53 | K52N | Forward | 5'-gactttagcttgggagaatggAATacccagatggag- 3' |
| | | Reverse | 5'-ctccatctgggtATTccattctcccaagctaaagtc- 3' |
| 54 | M55I | Forward | 5'-gggagaatggaaaacccagATTgaggagaccaag-3' |
| | | Reverse | 5'-cttggtctcctcAATctgggttttccattctccc-3' |
| 55 | E56Q | Forward | 5'-tggaaaacccagatgCAGgagaccaaggcac-3' |
| | | Reverse | 5'-gtgccttggtctcCTGcatctgggttttcca-3' |
| 56 | E56N | Forward | 5'-gaatggaaaacccagatgAATgagaccaaggcac-3' |
| | | Reverse | 5'-gtgccttggtctcATTcatctgggttttccattc-3' |
| 57 | E57Q | Forward | 5'-aaaacccagatggagCAGaccaaggcacagg-3' |
| | | Reverse | 5'-cctgtgccttggtCTGctccatctgggtttt-3' |
| 58 | E57N | Forward | 5'-gaaaacccagatggagAATaccaaggcacaggac-3' |
| | | Reverse | 5'-gtcctgtgccttggtATTctccatctgggttttc-3' |
| 59 | K59Q | Forward | 5'-cagatggaggagaccCAGgcacaggacattc-3' |
| | | Reverse | 5'-gaatgtcctgtgcCTGggtctcctccatctg-3' |
| 60 | K59N | Forward | 5'-cagatggaggagaccAATgcacaggacattctg-3' |
| | | Reverse | 5'-cagaatgtcctgtgcATTggtctcctccatctg-3' |
| 61 | A60S | Forward | 5'-gatggaggagaccaagAGCcaggacattctggg-3' |
| | | Reverse | 5'-cccagaatgtcctgGCTcttggtctcctccatc-3' |
| 62 | A60T | Forward | 5'-tggaggagaccaagACCcaggacattctgggag-3' |
| | | Reverse | 5'-ctcccagaatgtcctgGGTcttggtctcctcca-3' |
| 63 | D62Q | Forward | 5'-gagaccaaggcacagCAGattctgggagcagtg-3' |
| | | Reverse | 5'-cactgctcccagaatCTGctgtgccttggtctc-3' |
| 64 | D62N | Forward | 5'-gagaccaaggcacagAATattctgggagcag-3' |
| | | Reverse | 5'-ctgctcccagaatATTctgtgccttggtctc-3' |
| 65 | L64I | Forward | 5'-gaccaaggcacaggacattATTggagcagtgacc-3' |
| | | Reverse | 5'-ggtcactgctccAATaatgtcctgtgccttggtc-3' |
| 66 | G65S | Forward | 5'-gcacaggacattctgAGCgcagtgacccttctgc-3' |
| | | Reverse | 5'-gcagaagggtcactgcGCTcagaatgtcctgtgc-3' |
| 67 | A66S | Forward | 5'-caggacattctgggaAGCgtgacccttctgc-3' |
| | | Reverse | 5'-gcagaagggtcacGCTtcccagaatgtcctg-3' |
| 68 | A66T | Forward | 5'-acaggacattctgggaACCgtgacccttctgctgg- 3' |
| | | Reverse | 5'-ccagcagaagggtcacGGTtcccagaatgtcctgt- 3' |
| 69 | V67I | Forward | 5'-ggacattctgggagcaATTacccttctgctggag-3' |
| | | Reverse | 5'-ctccagcagaagggtAATtgctcccagaatgtcc-3' |
| 70 | V67T | Forward | 5'-ggacattctgggagcaACCacccttctgctggag-3' |
| | | Reverse | 5'-ctccagcagaagggtGGTtgctcccagaatgtcc-3' |
| 71 | L69I | Forward | 5'-gggagcagtgaccATTctgctggaggg-3' |
| | | Reverse | 5'-ccctccagcagTTAggtcactgctccc-3' |
| 72 | L70I | Forward | 5'-tctgggagcagtgacccttATTctggagggagtg-3' |
| | | Reverse | 5'-cactccctccagAATaagggtcactgctcccaga-3' |
| 73 | L71I | Forward | 5'-gcagtgacccttctgATTgagggagtgatggcag-3' |
| | | Reverse | 5'-ctgccatcactccctcAATcagaagggtcactgc-3' |
| 74 | E72Q | Forward | 5'-gacccttctgctgCAGggagtgatggc-3' |
| | | Reverse | 5'-gccatcactccCTGcagcagaagggtc-3' |
| 75 | E72N | Forward | 5'-gtgacccttctgctgAATggagtgatggcagcac-3' |
| | | Reverse | 5'-gtgctgccatcactccATTcagcagaagggtcac-3' |
| 76 | E72S | Forward | 5'-gtgacccttctgctgAGCggagtgatggcagc-3' |
| | | Reverse | 5'-gctgccatcactccGCTcagcagaagggtcac-3' |
| 77 | G73S | Forward | 5'-cccttctgctggagAGCgtgatggcagcacg-3' |
| | | Reverse | 5'-cgtgctgccatcacGCTctccagcagaaggg-3' |
| 78 | V74I | Forward | 5'-cttctgctggagggaATTatggcagcacgggga-3' |
| | | Reverse | 5'-tccccgtgctgccatAATtccctccagcagaag-3' |
| 79 | V74T | Forward | 5'-cttctgctggagggaACCatggcagcacgggg-3' |
| | | Reverse | 5'-ccccgtgctgccatGGTtccctccagcagaag-3' |
| 80 | M75I | Forward | 5'-ctggagggagtgATTgcagcacgggga-3' |
| | | Reverse | 5'-tccccgtgctgcAATcactccctccag-3' |
| 81 | A76S | Forward | 5'-ggagggagtgatgAGCgcacggggaca-3' |
| | | Reverse | 5'-tgtccccgtgcGCTcatcactccctcc-3' |
| 82 | A76T | Forward | 5'-ctggagggagtgatgACCgcacggggacaactg-3' |
| | | Reverse | 5'-cagttgtccccgtgcGGTcatcactccctccag-3' |
| 83 | A77S | Forward | 5'-agggagtgatggcaAGCcggggacaactg-3' |
| | | Reverse | 5'-cagttgtccccgGCTtgccatcactccct-3' |
| 84 | A77T | Forward | 5'-ctggagggagtgatggcaACCcggggacaac-3' |
| | | Reverse | 5'-gttgtccccgGGTtgccatcactccctccag-3' |
| 85 | R78Q | Forward | 5'-agtgatggcagcaCAGggacaactgggac-3' |
| | | Reverse | 5'-gtcccagttgtccCTGtgctgccatcact-3' |
| 86 | G79S | Forward | 5'-tgatggcagcacggAGCcaactgggacccac-3' |
| | | Reverse | 5'-gtgggtcccagttgGCTccgtgctgccatca-3' |
| 87 | L81I | Forward | 5'-gcagcacggggacaaATTggacccacttgcc-3' |
| | | Reverse | 5'-ggcaagtgggtccAATttgtccccgtgctgc-3' |
| 88 | L86I | Forward | 5'-tgggacccacttgcATTtcatccctcctg-3' |
| | | Reverse | 5'-caggagggatgaAATgcaagtgggtccca-3' |
| 89 | L89I | Forward | 5'-cttgcctctcatccATTctggggcagctt-3' |
| | | Reverse | 5'-aagctgccccagAATggatgagaggcaag-3' |
| 90 | L90I | Forward | 5'-gcctctcatccctcATTgggcagctttctggac-3' |
| | | Reverse | 5'-gtccagaaagctgcccAATgagggatgagaggc-3' |
| 91 | G91S | Forward | 5'-ctctcatccctcctgAGCcagctttctggacag-3' |
| | | Reverse | 5'-ctgtccagaaagctgGCTcaggagggatgagag-3' |
| 92 | L93I | Forward | 5'-ccctcctggggcagATTtctggacaggtc-3' |
| | | Reverse | 5'-gacctgtccagaAATctgccccaggaggg-3' |
| 93 | G95S | Forward | 5'-ctggggcagctttctAGCcaggtccgtctcctc-3' |
| | | Reverse | 5'-gaggagacggacctgGCTdgdddgctgccccdg-3' |
| 94 | V97I | Forward | 5'-cagctttctggacagATTcgtctcctccttg-3' |
| | | Reverse | 5'-caaggaggagacgAATctgtccagaaagctg-3' |
| 95 | V97T | Forward | 5'-gcagctttctggacagACCcgtctcctccttggg-3' |
| | | Reverse | 5'-cccaaggaggagacgGGTctgtccagaaagctgc-3' |
| 96 | R98Q | Forward | 5'-tttctggacaggtcCAGctcctccttggggcc-3' |
| | | Reverse | 5'-ggccccaaggaggagCTGgdcctgtccdgddd-3' |
| 97 | L99I | Forward | 5'-ctttctggacaggtccgtATTctccttggg-3' |
| | | Reverse | 5'-cccaaggagAATacggacctgtccagaaag-3' |
| 98 | L100I | Forward | 5'-caggtccgtctcATTcttggggccc-3' |
| | | Reverse | 5'-gggccccaagAATgagacggacctg-3' |
| 99 | L101I | Forward | 5'-gtccgtctcctcATTggggccctgc-3' |
| | | Reverse | 5'-gcagggccccAATgaggagacggac-3' |
| 100 | G102S | Forward | 5'-ggtccgtctcctccttAGCgccctgcag-3' |
| | | Reverse | 5'-ctgcdgggcGCTddggdggdgdcggdcc-3' |
| 101 | A103S | Forward | 5'-gtctcctccttgggAGCctgcagagcctcc-3' |
| | | Reverse | 5'-ggaggctctgcagGCTcccddggdggdgdc-3' |
| 102 | A103T | Forward | 5'-ctcctccttgggACCctgcagagcc-3' |
| | | Reverse | 5'-ggctctgcagGGAcccaaggaggag-3' |
| 103 | L104I | Forward | 5'-tcctccttggggccATTcagagcctccttgg-3' |
| | | Reverse | 5'-ccaaggaggctctgAATggccccaaggagga-3' |
| 104 | L107I | Forward | 5'-ggccctgcagagcATCcttggaaccca-3' |
| | | Reverse | 5'-tgggttccaagGATgctctgcagggcc-3' |
| 105 | L108I | Forward | 5'-ccctgcagagcctcATTggaacccagctt-3' |
| | | Reverse | 5'-aagctgggttccAATgaggctctgcaggg-3' |
| 106 | G109S | Forward | 5'-ggccctgcagagcctccttAGCacccagcttc-3' |
| | | Reverse | 5'-gaagctgggtGCTaaggaggctctgcagggcc-3' |
| 107 | L112I | Forward | 5'-tccttggaacccagATTcctccacagggc-3' |
| | | Reverse | 5'-gccctgtggaggAATctgggttccaagga-3' |
| 108 | Gl16S | Forward | 5'-gcttcctccacagAGCaggaccacagc-3' |
| | | Reverse | 5'-gctgtggtcctGCTctgtggaggaagc-3' |
| 109 | R117Q | Forward | 5'-gcttcctccacagggcCAGaccacagctcacaag-3' |
| | | Reverse | 5'-cttgtgagctgtggtCTGgccctgtggaggaagc-3' |
| 110 | A120S | Forward | 5'-ccacagggcaggaccacaAGCcacaaggatccc-3' |
| | | Reverse | 5'-gggatccttgtgGCTtgtggtcctgccctgtgg-3' |
| 111 | A120T | Forward | 5'-agggcaggaccacaACCcacaaggatccc-3' |
| | | Reverse | 5'-gggatccttgtgGGTtgtggtcctgccct-3' |
| 112 | H121N | Forward | 5'-gggcaggaccacagctAATaaggatcccaa-3' |
| | | Reverse | 5'-ttgggatccttATTagctgtggtcctgccc-3' |
| 113 | H121Q | Forward | 5'-caggaccacagctCAGaaggatcccaatgcc-3' |
| | | Reverse | 5'-ggcattgggatccttCTGagctgtggtcctg-3' |
| 114 | K122N | Forward | 5'-ggaccacagctcacAATgatcccaatgccatct-3' |
| | | Reverse | 5'-agatggcattgggatcATTgtgagctgtggtcc-3' |
| 115 | K122Q | Forward | 5'-ggaccacagctcacCAGgatcccaatgcc-3' |
| | | Reverse | 5'-ggcattgggatcCTGgtgagctgtggtcc-3' |
| 116 | D123Q | Forward | 5'-gaccacagctcacaagCAGcccaatgccatcttc-3' |
| | | Reverse | 5'-gaagatggcattgggCTGcttgtgagctgtggtc-3' |
| 117 | D123N | Forward | 5'-gaccacagctcacaagAATcccaatgccatcttc-3' |
| | | Reverse | 5'-gaagatggcattgggATTcttgtgagctgtggtc-3' |
| 118 | A126S | Forward | 5'-ctcacaaggatcccaatAGCatcttcctgagctt-3' |
| | | Reverse | 5'-aagctcaggaagatGCTattgggatccttgtgag-3' |
| 119 | A126T | Forward | 5'-acagctcacaaggatcccaatACCatcttcctga-3' |
| | | Reverse | 5'-tcaggaagatGGTattgggatccttgtgagctgt-3' |
| 120 | F128I | Forward | 5'-ggatcccaatgccatcATCctgagcttccaaca-3' |
| | | Reverse | 5'-tgttggaagctcagGATgatggcattgggatcc-3' |
| 121 | L129I | Forward | 5'-cccaatgccatcttcATCagcttccaacacctgc-3' |
| | | Reverse | 5'-gcaggtgttggaagctGATgaagatggcattggg-3' |
| 122 | L129V | Forward | 5'-cccaatgccatcttcGTGagcttccaacacctgc-3' |
| | | Reverse | 5'-gcaggtgttggaagctCACgaagatggcattggg-3' |
| 123 | F131I | Forward | 5'-gccatcttcctgagcATCcaacacctgctcc-3' |
| | | Reverse | 5'-ggagcaggtgttgGATgctcaggaagatggc-3' |
| 124 | H133N | Forward | 5'-gccatcttcctgagcttccaaAATctgctccga-3' |
| | | Reverse | 5'-tcggagcagATTttggaagctcaggaagatggc-3' |
| 125 | H133Q | Forward | 5'-cctgagcttccaaCAGctgctccgaggaaag-3' |
| | | Reverse | 5'-ctttcctcggagcagCTGttggaagctcagg-3' |
| 126 | L134I | Forward | 5'-ctgagcttccaacacATTctccgaggaaaggtgc-3' |
| | | Reverse | 5'-gcacctttcctcggagAATgtgttggaagctcag-3' |
| 127 | L135I | Forward | 5'-agcttccaacacctgATCcgaggaaaggtgc-3' |
| | | Reverse | 5'-gcacctttcctcgGATcaggtgttggaagct-3' |
| 128 | R136Q | Forward | 5'-ttccaacacctgctcCAGggaaaggtgcgtttc-3' |
| | | Reverse | 5'-gaaacgcacctttccCTGgagcaggtgttggaa-3' |
| 129 | G137S | Forward | 5'-caacacctgctccgaAGCaaggtgcgtttcctga-3' |
| | | Reverse | 5'-tcaggaaacgcaccttGCTtcggagcaggtgttg-3' |
| 130 | K138N | Forward | 5'-acacctgctccgaggaAATgtgcgtttcctg-3' |
| | | Reverse | 5'-caggaaacgcacATTtcctcggagcaggtgt-3' |
| 131 | K138Q | Forward | 5'-cacctgctccgaggaCAGgtgcgtttcc-3' |
| | | Reverse | 5'-ggaaacgcacCTGtcctcggagcaggtg-3' |
| 132 | K138T | Forward | 5'-cacctgctccgaggaACCgtgcgtttcc-3' |
| | | Reverse | 5'-ggaaacgcacGGTtcctcggagcaggtg-3' |
| 133 | V139I | Forward | 5'-ctgctccgaggaaagATCcgtttcctgatgcttg-3' |
| | | Reverse | 5'-caagcatcaggaaacgGATctttcctcggagcag-3' |
| 134 | V139T | Forward | 5'-ctgctccgaggaaagACCcgtttcctgatgcttg-3' |
| | | Reverse | 5'-caagcatcaggaaacgGGTctttcctcggagcag-3' |
| 135 | R140Q | Forward | 5'-ctccgaggaaaggtgCAGttcctgatgcttgtag-3' |
| | | Reverse | 5'-ctacaagcatcaggaaCTGcacctttcctcggag-3' |
| 136 | F141I | Forward | 5'-gctccgaggaaaggtgcgtATCctgatgct-3' |
| | | Reverse | 5'-agcatcagGATacgcacctttcctcggagc-3' |
| 137 | F141S | Forward | 5'-gctccgaggaaaggtgcgtAGCctgatgct-3' |
| | | Reverse | 5'-agcatcagGCTacgcacctttcctcggagc-3' |
| 138 | L142I | Forward | 5'-aaaggtgcgtttcATCatgcttgtaggagggtcc-3' |
| | | Reverse | 5'-ggaccctcctacaagcatGATgaaacgcaccttt-3' |
| 139 | M143I | Forward | 5'-ggtgcgtttcctgATCcttgtaggagggtcc-3' |
| | | Reverse | 5'-ggaccctcctacaagGATcaggaaacgcacc-3' |
| 140 | M143N | Forward | 5'-ggtgcgtttcctgAATcttgtaggagggtccacc-3' |
| | | Reverse | 5'-ggtggaccctcctacaagATTcaggaaacgcacc-3' |
| 141 | L144I | Forward | 5'-ggtgcgtttcctgatgATTgtaggagggtccac-3' |
| | | Reverse | 5'-gtggaccctcctacAATcatcaggaaacgcacc-3' |
| 142 | V145I | Forward | 5'-aggtgcgtttcctgatgcttATCggagggtcc-3' |
| | | Reverse | 5'-ggaccctccGATaagcatcaggaaacgcacct-3' |
| 143 | V145T | Forward | 5'-gtgcgtttcctgatgcttACCggagggtccaccc-3' |
| | | Reverse | 5'-gggtggaccctccGGTaagcatcaggaaacgcac-3' |
| 144 | G146S | Forward | 5'-gtgcgtttcctgatgcttgtaAGCgggtccaccc-3' |
| | | Reverse | 5'-gggtggacccGCTtacaagcatcaggaaacgcac-3' |
| 145 | G147S | Forward | 5'-gtttcctgatgcttgtaggaAGCtccaccctctg-3' |
| | | Reverse | 5'-cagagggtggaGCTtcctacaagcatcaggaaac-3' |
| 146 | L150I | Forward | 5'-ggtccaccATCtgcgtgaggtaactcgagcgg-3' |
| | | Reverse | 5'-ccgctcgagttacctcacgcaGATggtggaccc-3' |
| 147 | V152I | Forward | 5'-ggtccaccctctgcATCaggtaactcgagcgg-3' |
| | | Reverse | 5'-ccgctcgagttacctGATgcagagggtggaccc-3' |
| 148 | V152T | Forward | 5'-gggtccaccctctgcACCaggtaactcgagcgg-3' |
| | | Reverse | 5'-ccgctcgagttacctGGTgcagagggtggaccc-3' |
| 149 | K138S | Forward | 5'-cacctgctccgaggaAGCgtgcgtttcc-3' |
| | | Reverse | 5'-ggaaacgcacGCTtcctcggagcaggtg-3' |

### [EXAMPLE 9] Expression of TP01-153 and TPO1-153 Variants in HEK293 cells

The mammalian expression vector pcDNA3.3-GH-TPO1-153 cloned in Example 3 and their variants constructed in Example 8 were expressed in HEK293.

In brief, after being isolated, each variant plasmid was dissolved at a concentration of 500 ng/µL. HEK293 cells were maintained in a DMEM medium including 10% FBS and seeded at a density of 200,000 cells/well on 6-well plates one day before transfection. Next day, 4 µg of DNA and 2.5 µL of Enhancer-Q were added to 50 µL of OPTI-MEM per well and incubated at room temperature for 15 min. To each well was added 2 µL of WelFect-Ex, followed by incubation at room temperature for 10 min. After removal of the medium, 1 mL of OPTI-MEM was added to each well. A mixture of DNA and liposomes was introduced into cells and incubated for 4 hours, after which 10% FBS supplemented DMEM was replaced with a fresh one. The cells were incubated for 72 hours, and the supernatant was harvested.

### [EXAMPLE 10] Quantification of TPO1-153 and TPO1-153 Variant Proteins Expressed in HEK293 cells

Expression yields of TPO1-153 and each variant were measured using an ELISA kit (Peprotech, Cat. No: 900-K44).

In detail, the assay diluent RD1-1 (Cat. No: 900-K44) was added in an amount of 50 µL per well of an anti-TPO monoclonal antibody-coated 96-well plates to which the standard protein TPO174 (Peprotech, Cat. No: 900-K44) or each sample was then added in an amount of 200 µL per well. The plates were sealed with vinyl tape and incubated at room temperature for 3 hours. Subsequently, the plates were washed three times with wash buffer (40mM Tris (pH7.5), 0.3M NaCl, 2.7mM KC1, 0.05% Tween20) and treated with the TPO Conjugate (goat anti-TPO IgG-HRP conjugate (Cat. No: 900-K44)). Sealing with vinyl tape was followed by incubation at room temperature for 1 hour. Again, the plates were washed three times with wash buffer, followed by the addition of 200 µL of a substrate solution to each well. After incubation for 30 min, absorbance at 450 nm of each well was read on a microplate reader. With normalization to the standard curve, the concentrations of TPO1-153 and TPO1-153 variants in samples were calculated. The remaining samples were stored at -80°C.

Expression levels of TPO1-153 and TPO1-153 variants determined by ELISA are summarized in Table 4, below.

**TABLE 4 Expression Levels of TPO1-153 and TPO1-153 Variants**

| Variant No. | Expression Level (ng/ml) | Variant No. | Expression Level (ng/ml) |
|---|---|---|---|
| TPO1-153 | 500 | TPO1-153-75 | 174 |
| TPO1-153-1 | 418 | TPO1-153-76 | 54 |
| TPO1-153-2 | 649 | TPO1-153-77 | 415 |
| TPO1-153-3 | 218 | TPO1-153-78 | 737 |
| TPO1-153-4 | 389 | TPO1-153-79 | 140 |
| TPO1-153-5 | 278 | TPO1-153-80 | 221 |
| TPO1-153-6 | 217 | TPO1-153-81 | 246 |
| TPO1-153-7 | 181 | TPO1-153-82 | 113 |
| TPO1-153-8 | 260 | TPO1-153-83 | ND |
| TPO1-153-9 | 377 | TPO1-153-84 | ND |
| TPO1-153-10 | 226 | TPO1-153-85 | 455 |
| TPO1-153-11 | 333 | TPO1-153-86 | 494 |
| TPO1-153-12 | 285 | TPO1-153-87 | 121 |
| TPO1-153-13 | 262 | TPO1-153-88 | 160 |
| TPO1-153-14 | 277 | TPO1-153-89 | 176 |
| TPO1-153-15 | 404 | TPO1-153-90 | 5 |
| TPO1-153-16 | 138 | TPO1-153-91 | 105 |
| TPO1-153-17 | 147 | TPO1-153-92 | 14 |
| TPO1-153-18 | 121 | TPO1-153-93 | 314 |
| TPO1-153-19 | 250 | TPO1-153-94 | 383 |
| TPO1-153-20 | 412 | TPO1-153-95 | 37 |
| TPO1-153-21 | 442 | TPO1-153-96 | 535 |
| TPO1-153-22 | 88 | TPO1-153-97 | 509 |
| TPO1-153-23 | 334 | TPO1-153-98 | 68 |
| TPO1-153-24 | 88 | TPO1-153-99 | 162 |
| TPO1-153-25 | 315 | TPO1-153-100 | 95 |
| TPO1-153-26 | 539 | TPO1-153-101 | 522 |
| TPO1-153-27 | 205 | TPO1-153-102 | 436 |
| TPO1-153-28 | 198 | TPO1-153-103 | 154 |
| TPO1-153-29 | 148 | TPO1-153-104 | 594 |
| TPO1-153-30 | 417 | TPO1-153-105 | 17 |
| TPO1-153-31 | 24 | TPO1-153-106 | 145 |
| TPO1-153-32 | 361 | TPO1-153-107 | 324 |
| TPO1-153-33 | 407 | TPO1-153-108 | 199 |
| TPO1-153-34 | 115 | TPO1-153-109 | 286 |
| TPO1-153-35 | 229 | TPO1-153-110 | 232 |
| TPO1-153-36 | 12 | TPO1-153-111 | 197 |
| TPO1-153-37 | 224 | TPO1-153-112 | 214 |
| TPO1-153-38 | 44 | TPO1-153-113 | 218 |
| TPO1-153-39 | 226 | TPO1-153-114 | 383 |
| TPO1-153-40 | 180 | TPO1-153-115 | 297 |
| TPO1-153-41 | 202 | TPO1-153-116 | 11 |
| TPO1-153-42 | 560 | TPO1-153-117 | 35 |
| TPO1-153-43 | 66 | TPO1-153-118 | 140 |
| TPO1-153-44 | 547 | TPO1-153-119 | 63 |
| TPO1-153-45 | 328 | TPO1-153-120 | 22 |
| TPO1-153-46 | 282 | TPO1-153-121 | 190 |
| TPO1-153-47 | 584 | TPO1-153-122 | 225 |
| TPO1-153-48 | 191 | TPO1-153-123 | ND |
| TPO1-153-49 | 49 | TPO1-153-124 | 13 |
| TPO1-153-50 | 95 | TPO1-153-125 | 515 |
| TPO1-153-51 | 169 | TPO1-153-126 | 489 |
| TPO1-153-52 | 276 | TPO1-153-127 | 232 |
| TPO1-153-53 | 635 | TPO1-153-128 | 58 |
| TPO1-153-54 | 271 | TPO1-153-129 | 421 |
| TPO1-153-55 | 276 | TPO1-153-130 | 754 |
| TPO1-153-56 | 1295 | TPO1-153-131 | 273 |
| TPO1-153-57 | 203 | TPO1-153-132 | 1096 |
| TPO1-153-58 | 150 | TPO1-153-133 | 1118 |
| TPO1-153-59 | 395 | TPO1-153-134 | 588 |
| TPO1-153-60 | 432 | TPO1-153-135 | 105 |
| TPO1-153-61 | 217 | TPO1-153-136 | 326 |
| TPO1-153-62 | 186 | TPO1-153-137 | 208 |
| TPO1-153-63 | 108 | TPO1-153-138 | 587 |
| TPO1-153-64 | 107 | TPO1-153-139 | 87 |
| TPO1-153-65 | 214 | TPO1-153-140 | 163 |
| TPO1-153-66 | 170 | TPO1-153-141 | 471 |
| TPO1-153-67 | 304 | TPO1-153-142 | 83 |
| TPO1-153-68 | 107 | TPO1-153-143 | 311 |
| TPO1-153-69 | 154 | TPO1-153-144 | 975 |
| TPO1-153-70 | 392 | TPO1-153-145 | 331 |
| TPO1-153-71 | 301 | TPO1-153-146 | 294 |
| TPO1-153-72 | 256 | TPO1-153-147 | 146 |
| TPO1-153-73 | 17 | TPO1-153-148 | 233 |
| TPO1-153-74 | 309 | TPO1-153-149 | 523 |

| | | | |
|---|---|---|---|
| *ND: Not Detected | | | |

### [EXAMPLE 11] In vitro Assay of TPO1-153 and TPO1-153 Variants for Biological Activity in Terms of STAT5 Phosphorylation

STAT5, located in M-o7e cells, is phosphorylated in the presence of native TPO. Hence, STAT5 phosphorylation can be a barometer indicating the biological activity of the TPO1-153 variants compared to that of TPO (FIG. 5).

The activity of TPO1-153 and TPO1-153 variants was assayed by measuring STAT5 phosphorylation as follows. One mL of M-o7e cells (0.5-2x10⁶ cells/mL) cultured in RPMI1640 media containing 10% FBS and 10 ng/ml GM-CSF was seeded at a density of 0.5x10⁶ cells/ml into 6-well plates. The cells were washed with 10% FBS-supplemented RPMI1640 media to remove GM-CSF and then incubated for 50 hours. Culture media in which the expression levels of the TPO1-153 or the TPO1-153 variants were determined after transient expression from the transfected HEK293 cells, were diluted in RPMI1640 to adjust the concentrations of the proteins to 100 pg/mL, and 100 µL of the dilution was added to the cell culture and uniformly dispersed by gentle shaking. Conditioned media in which TPO153 was not expressed were also treated in the same manner as above. After incubation for 30 min, the cells were harvested in 1.5 mL tubes by centrifugation (15,000 rpm, 5 min).

The cell pellets were resuspended in PBS stored at 4°C and centrifuged again. After complete removal of PBS, 100 µL of lysis buffer (Pierce, 89900) was added to each tube and vortexed for 20 sec. Incubation for 20 min on ice, followed by centrifugation to cause the cell debris to pelletize. The protein level of the supernatant was quantitatively analyzed using the Bradford method, and the STAT5 phosphorylation of the proteins was assayed using an ELISA kit (USbiological, Cat. No: 57969-95).

The biological activities of the TPO1-153 variants in terms of STAT5 phosphorylation are given in Table 5, below.

**TABLE 5 Biological Activity of TPO1-153 and TPO1-153 Variants**

| Variant No. | Activity Relative to TPO1-153 | Variant No. | Activity Relative to TPO1-153 |
|---|---|---|---|
| TPO1-153-1 | 132% | TPO1-153-76 | 87% |
| TPO1-153-2 | 81% | TPO1-153-77 | 164% |
| TPO1-153-3 | 85% | TPO1-153-78 | 131% |
| TPO1-153-4 | 103% | TPO1-153-79 | 189% |
| TPO1-153-5 | 60% | TPO1-153-80 | 151% |
| TPO1-153-6 | 60% | TPO1-153-81 | 156% |
| TPO1-153-7 | 189% | TPO1-153-82 | 96% |
| TPO1-153-8 | 84% | TPO1-153-83 | N/A |
| TPO1-153-9 | 100% | TPO1-153-84 | N/A |
| TPO1-153-10 | 77% | TPO1-153-85 | 59% |
| TPO1-153-11 | 182% | TPO1-153-86 | 155% |
| TPO1-153-12 | 33% | TPO1-153-87 | 219% |
| TPO1-153-13 | 29% | TPO1-153-88 | 112% |
| TPO1-153-14 | 112% | TPO1-153-89 | ND |
| TPO1-153-15 | 122% | TPO1-153-90 | ND |
| TPO1-153-16 | 123% | TPO1-153-91 | 104% |
| TPO1-153-17 | 169% | TPO1-153-92 | 44% |
| TPO1-153-18 | 163% | TPO1-153-93 | 142% |
| TPO1-153-19 | 122% | TPO1-153-94 | 136% |
| TPO1-153-20 | ND | TPO1-153-95 | 61% |
| TPO1-153-21 | 104% | TPO1-153-96 | 58% |
| TPO1-153-22 | 3% | TPO1-153-97 | 180% |
| TPO1-153-23 | 157% | TPO1-153-98 | 2% |
| TPO1-153-24 | 151% | TPO1-153-99 | 107% |
| TPO1-153-25 | 123% | TPO1-153-100 | 34% |
| TPO1-153-26 | 76% | TPO1-153-101 | 123% |
| TPO1-153-27 | 31% | TPO1-153-102 | 87% |
| TPO1-153-28 | 85% | TPO1-153-103 | 157% |
| TPO1-153-29 | 76% | TPO1-153-104 | 117% |
| TPO1-153-30 | 106% | TPO1-153-105 | 94% |
| TPO1-153-31 | 55% | TPO1-153-106 | 133% |
| TPO1-153-32 | 152% | TPO1-153-107 | 93% |
| TPO1-153-33 | 122% | TPO1-153-108 | 103% |
| TPO1-153-34 | 122% | TPO1-153-109 | 85% |
| TPO1-153-35 | 147% | TPO1-153-110 | 87% |
| TPO1-153-36 | 34% | TPO1-153-111 | 113% |
| TPO1-153-37 | 193% | TPO1-153-112 | 199% |
| TPO1-153-38 | 124% | TPO1-153-113 | 100% |
| TPO1-153-39 | 186% | TPO1-153-114 | 170% |
| TPO1-153-40 | 110% | TPO1-153-115 | 175% |
| TPO1-153-41 | 86% | TPO1-153-116 | 28% |
| TPO1-153-42 | 101% | TPO1-153-117 | 105% |
| TPO1-153-43 | -1% | TPO1-153-118 | 88% |
| TPO1-153-44 | 6% | TPO1-153-119 | 107% |
| TPO1-153-45 | 94% | TPO1-153-120 | 67% |
| TPO1-153-46 | 126% | TPO1-153-121 | 131% |
| TPO1-153-47 | 79% | TPO1-153-122 | 125% |
| TPO1-153-48 | 94% | TPO1-153-123 | N/A |
| TPO1-153-49 | 111% | TPO1-153-124 | 130% |
| TPO1-153-50 | 83% | TPO1-153-125 | 152% |
| TPO1-153-51 | 19% | TPO1-153-126 | 111% |
| TPO1-153-52 | 95% | TPO1-153-127 | 139% |
| TPO1-153-53 | 94% | TPO1-153-128 | 76% |
| TPO1-153-54 | 139% | TPO1-153-129 | 2% |
| TPO1-153-55 | 133% | TPO1-153-130 | 1% |
| TPO1-153-56 | 138% | TPO1-153-131 | 18% |
| TPO1-153-57 | 207% | TPO1-153-132 | -1% |
| TPO1-153-58 | 180% | TPO1-153-133 | 119% |
| TPO1-153-59 | 118% | TPO1-153-134 | 90% |
| TPO1-153-60 | 90% | TPO1-153-135 | 5% |
| TPO1-153-61 | 109% | TPO1-153-136 | 74% |
| TPO1-153-62 | 211% | TPO1-153-137 | -1% |
| TPO1-153-63 | 170% | TPO1-153-138 | 128% |
| TPO1-153-64 | 108% | TPO1-153-139 | 183% |
| TPO1-153-65 | 100% | TPO1-153-140 | 6% |
| TPO1-153-66 | 189% | TPO1-153-141 | 8% |
| TPO1-153-67 | 161% | TPO1-153-142 | 127% |
| TPO1-153-68 | 116% | TPO1-153-143 | 147% |
| TPO1-153-69 | 103% | TPO1-153-144 | 131% |
| TPO1-153-70 | 142% | TPO1-153-145 | 106% |
| TPO1-153-71 | 147% | TPO1-153-146 | 124% |
| TPO1-153-72 | 122% | TPO1-153-147 | 149% |
| TPO1-153-73 | 72% | TPO1-153-148 | 102% |
| TPO1-153-74 | 104% | TPO1-153-149 | 36% |
| TPO1-153-75 | 97% | | |

| | | | |
|---|---|---|---|
| *ND: Not Detected, N/A: Not Available | | | |

### [EXAMPLE 12] Determination of Resistance of TPO1-153 and TPO1-153 Variants to Proteases

To select TPO1-153 variants resistant to proteases, variants with increased half life were obtained after treatment with 10 different proteases. Experimental results for the resistance of the representative variants TPO1-153-132, TPO1-153-67 and TPO1-153-87 to a protease mix are depicted in FIG. 8.

Details are given of the experiment, below.

The expression medium of TPO1-153 containing a total protein amount of 20 µg was mixed with 30 µL of 500 mM Tris-HCl (pH7.4) and H₂O in the valance amount to 300 µL. As a sample at 0 min, 30 µL of the dilution was taken and mixed with 2 µL of a protease inhibitor (Roche, Cat. No:11836170001) before storage at -80°C. To the remaining sample, each of the 10 proteases trypsin, chymotrypsin, thrombin, elastases, Arg-C endopepdidase, Asn-N endopeptidase, Glu-C endopeptidase, Pro-C endopeptidase, Lys-C endopeptidase, and carboxypeptidase Y, all being commercially available from Sigma, was added in an amount of 1.8 µL corresponding to 1 % of the total protein amount and allowed to react at 25°C and 30°C. After reaction for 5 min, 10 min, 20 min, 40 min, 60 min, 80 min and 100 min, 32 µL of a sample was taken from the reaction mixture and mixed with 2 µL of a protease inhibitor before storage at - 80°C.

After reaction, the levels of undigested proteins in samples were quantitatively analyzed using ELISA to determine half lives of the TPO variants in duplicate. The average half lives of the TPO variants are represented as a percentage of those of TPO1-153 in Tables 6 and 7.

**TABLE 6 Resistance of TPO1-153 Variants to Proteases (30°C)**

| Variant No. | Resistance Relative to TPO1-153 | Variant No. | Resistance Relative to TPO1-153 |
|---|---|---|---|
| TPO1-153-1 | 110% | TPO1-153-76 | 74% |
| TPO1-153-2 | 136% | TPO1-153-77 | 103% |
| TPO1-153-3 | 75% | TPO1-153-78 | 117% |
| TPO1-153-4 | 88% | TPO1-153-79 | 111% |
| TPO1-153-5 | 72% | TPO1-153-80 | 104% |
| TPO1-153-6 | 80% | TPO1-153-81 | 118% |
| TPO1-153-7 | 125% | TPO1-153-82 | 96% |
| TPO1-153-8 | 99% | TPO1-153-83 | N/A |
| TPO1-153-9 | 103% | TPO1-153-84 | N/A |
| TPO1-153-10 | 77% | TPO1-153-85 | 71% |
| TPO1-153-11 | 87% | TPO1-153-86 | 131% |
| TPO1-153-12 | 135% | TPO1-153-87 | 161% |
| TPO1-153-13 | 108% | TPO1-153-88 | 91% |
| TPO1-153-14 | 75% | TPO1-153-89 | ND |
| TPO1-153-15 | 117% | TPO1-153-90 | ND |
| TPO1-153-16 | 142% | TPO1-153-91 | 76% |
| TPO1-153-17 | 90% | TPO1-153-92 | ND |
| TPO1-153-18 | 106% | TPO1-153-93 | 90% |
| TPO1-153-19 | 93% | TPO1-153-94 | 93% |
| TPO1-153-20 | 205% | TPO1-153-95 | ND |
| TPO1-153-21 | 136% | TPO1-153-96 | 76% |
| TPO1-153-22 | 192% | TPO1-153-97 | 133% |
| TPO1-153-23 | 109% | TPO1-153-98 | 45% |
| TPO1-153-24 | 116% | TPO1-153-99 | 79% |
| TPO1-153-25 | 82% | TPO1-153-100 | 104% |
| TPO1-153-26 | 124% | TPO1-153-101 | 83% |
| TPO1-153-27 | 107% | TPO1-153-102 | 110% |
| TPO1-153-28 | 92% | TPO1-153-103 | 123% |
| TPO1-153-29 | 79% | TPO1-153-104 | 104% |
| TPO1-153-30 | 157% | TPO1-153-105 | 91% |
| TPO1-153-31 | ND | TPO1-153-106 | 108% |
| TPO1-153-32 | 80% | TPO1-153-107 | 67% |
| TPO1-153-33 | 73% | TPO1-153-108 | 76% |
| TPO1-153-34 | 82% | TPO1-153-109 | 97% |
| TPO1-153-35 | 86% | TPO1-153-110 | 64% |
| TPO1-153-36 | ND | TPO1-153-111 | 56% |
| TPO1-153-37 | 108% | TPO1-153-112 | 89% |
| TPO1-153-38 | 70% | TPO1-153-113 | 81% |
| TPO1-153-39 | 127% | TPO1-153-114 | 93% |
| TPO1-153-40 | 96% | TPO1-153-115 | 132% |
| TPO1-153-41 | 97% | TPO1-153-116 | ND |
| TPO1-153-42 | 117% | TPO1-153-117 | 41% |
| TPO1-153-43 | 78% | TPO1-153-118 | 80% |
| TPO1-153-44 | 201% | TPO1-153-119 | 50% |
| TPO1-153-45 | 157% | TPO1-153-120 | 68% |
| TPO1-153-46 | 98% | TPO1-153-121 | 112% |
| TPO1-153-47 | 52% | TPO1-153-122 | 57% |
| TPO1-153-48 | 112% | TPO1-153-123 | N/A |
| TPO1-153-49 | 99% | TPO1-153-124 | 109% |
| TPO1-153-50 | 81% | TPO1-153-125 | 105% |
| TPO1-153-51 | 102% | TPO1-153-126 | 97% |
| TPO1-153-52 | 130% | TPO1-153-127 | 113% |
| TPO1-153-53 | 173% | TPO1-153-128 | 127% |
| TPO1-153-54 | 73% | TPO1-153-129 | 106% |
| TPO1-153-55 | 73% | TPO1-153-130 | 109% |
| TPO1-153-56 | 95% | TPO1-153-131 | 290% |
| TPO1-153-57 | 102% | TPO1-153-132 | 700% |
| TPO1-153-58 | 117% | TPO1-153-133 | 218% |
| TPO1-153-59 | 84% | TPO1-153-134 | 68% |
| TPO1-153-60 | 125% | TPO-153-135 | 172% |
| TPO1-153-61 | 76% | TPO1-153-136 | 94% |
| TPO1-153-62 | 102% | TPO1-153-137 | 157% |
| TPO1-153-63 | 106% | TPO1-153-138 | 97% |
| TPO1-153-64 | 78% | TPO1-153-139 | 128% |
| TPO1-153-65 | 72% | TPO1-153-140 | 111% |
| TPO1-153-66 | 104% | TPO1-153-141 | 80% |
| TPO1-153-67 | 126% | TPO1-153-142 | 85% |
| TPO1-153-68 | 68% | TPO1-153-143 | 87% |
| TPO1-153-69 | 78% | TPO1-153-144 | 85% |
| TPO1-153-70 | 163% | TPO1-153-145 | 87% |
| TPO1-153-71 | 104% | TPO1-153-146 | 133% |
| TPO1-153-72 | 84% | TPO1-153-147 | 91% |
| TPO1-153-73 | 51% | TPO1-153-148 | 111% |
| TPO1-153-74 | 99% | TPO1-153-149 | 505% |
| TPO1-153-75 | 90% | | |

| | | | |
|---|---|---|---|
| *ND: Not Detected | | | |

**TABLE 7 Resistance of TPO1-153 Variants to Proteases (25°C)**

| Variant No. | Resistance Relative to TPO1-153 | Variant No. | Resistance Relative to TPO1-153 |
|---|---|---|---|
| TPO1-153-1 | 95% | TPO1-153-76 | 95% |
| TPO1-153-2 | 149% | TPO1-153-77 | 116% |
| TPO1-153-3 | 84% | TPO1-153-78 | 135% |
| TPO1-153-4 | 104% | TPO1-153-79 | 106% |
| TPO1-153-5 | 72% | TPO1-153-80 | 121% |
| TPO1-153-6 | 85% | TPO1-153-81 | 181% |
| TPO1-153-7 | 123% | TPO1-153-82 | 94% |
| TPO1-153-8 | 90% | TPO1-153-83 | N/A |
| TPO1-153-9 | 82% | TPO1-153-84 | N/A |
| TPO1-153-10 | 58% | TPO1-153-85 | 69% |
| TPO1-153-11 | 112% | TPO1-153-86 | 118% |
| TPO1-153-12 | 77% | TPO1-153-87 | 111% |
| TPO1-153-13 | 97% | TPO1-153-88 | 78% |
| TPO1-153-14 | 75% | TPO1-153-89 | ND |
| TPO1-153-15 | 113% | TPO1-153-90 | ND |
| TPO1-153-16 | 172% | TPO1-153-91 | 69% |
| TPO1-153-17 | 106% | TPO1-153-92 | ND |
| TPO1-153-18 | 102% | TPO1-153-93 | 89% |
| TPO1-153-19 | 75% | TPO1-153-94 | 85% |
| TPO1-153-20 | 156% | TPO1-153-95 | ND |
| TPO1-153-21 | 78% | TPO1-153-96 | 72% |
| TPO1-153-22 | 225% | TPO1-153-97 | 92% |
| TPO1-153-23 | 122% | TPO1-153-98 | 41% |
| TPO1-153-24 | 141% | TPO1-153-99 | 94% |
| TPO1-153-25 | 90% | TPO1-153-100 | 94% |
| TPO1-153-26 | 86% | TPO1-153-101 | 95% |
| TPO1-153-27 | 105% | TPO1-153-102 | 101% |
| TPO1-153-28 | 78% | TPO1-153-103 | 83% |
| TPO1-153-29 | 91% | TPO1-153-104 | 74% |
| TPO1-153-30 | 159% | TPO1-153-105 | 97% |
| TPO1-153-31 | ND | TPO1-153-106 | 113% |
| TPO1-153-32 | 78% | TPO1-153-107 | 85% |
| TPO1-153-33 | 92% | TPO1-153-108 | 79% |
| TPO1-153-34 | 110% | TPO1-153-109 | 110% |
| TPO1-153-35 | 101% | TPO1-153-110 | 84% |
| TPO1-153-36 | ND | TPO1-153-111 | 67% |
| TPO1-153-37 | 138% | TPO1-153-112 | 108% |
| TPO1-153-38 | 62% | TPO1-153-113 | 70% |
| TPO1-153-39 | 103% | TPO1-153-114 | 100% |
| TPO1-153-40 | 83% | TPO1-153-115 | 131% |
| TPO1-153-41 | 110% | TPO1-153-116 | ND |
| TPO1-153-42 | 95% | TPO1-153-117 | 40% |
| TPO1-153-43 | 89% | TPO1-153-118 | 87% |
| TPO1-153-44 | 103% | TPO1-153-119 | 58% |
| TPO1-153-45 | 123% | TPO1-153-120 | 73% |
| TPO1-153-46 | 74% | TPO1-153-121 | 119% |
| TPO1-153-47 | 50% | TPO1-153-122 | 76% |
| TPO1-153-48 | 82% | TPO1-153-123 | N/A |
| TPO1-153-49 | 66% | TPO1-153-124 | 74% |
| TPO1-153-50 | 82% | TPO1-153-125 | 99% |
| TPO1-153-51 | 106% | TPO1-153-126 | 100% |
| TPO1-153-52 | 94% | TPO1-153-127 | 104% |
| TPO1-153-53 | 302% | TPO1-153-128 | 131% |
| TPO1-153-54 | 88% | TPO1-153-129 | 158% |
| TPO1-153-55 | 101% | TPO1-153-130 | 112% |
| TPO1-153-56 | 147% | TPO1-153-131 | 197% |
| TPO1-153-57 | 92% | TPO1-153-132 | 1087% |
| TPO1-153-58 | 113% | TPO1-153-133 | 146% |
| TPO1-153-59 | 68% | TPO1-153-134 | 75% |
| TPO1-153-60 | 183% | TPO1-153-135 | 195% |
| TPO1-153-61 | 92% | TPO1-153-136 | 109% |
| TPO1-153-62 | 83% | TPO1-153-137 | 225% |
| TPO1-153-63 | 106% | TPO1-153-138 | 117% |
| TPO1-153-64 | 93% | TPO1-153-139 | 118% |
| TPO1-153-65 | 79% | TPO1-153-140 | 101% |
| TPO1-153-66 | 106% | TPO1-153-141 | 75% |
| TPO1-153-67 | 245% | TPO1-153-142 | 88% |
| TPO1-153-68 | 64% | TPO1-153-143 | 117% |
| TPO1-153-69 | 73% | TPO1-153-144 | 57% |
| TPO1-153-70 | 142% | TPO1-153-145 | 73% |
| TPO1-153-71 | 113% | TPO1-153-146 | 73% |
| TPO1-153-72 | 77% | TPO1-153-147 | 92% |
| TPO1-153-73 | 67% | TPO1-153-148 | 124% |
| TPO1-153-74 | 85% | TPO1-153-149 | 554% |
| TPO1-153-75 | 72% | | |

| | | | |
|---|---|---|---|
| *ND: Not Detected, N/A: Not Available | | | |

Compared to TPO1-153, as can be seen in Tables 6 and 7, the TPO1-153 variant polypeptides of the present invention exhibited higher resistance to various proteases present in the gastrointestinal tract, cells and blood by at least 50%, 100%, 150%, 200% and even 1,000%.

### [EXAMPLE 13] Design of TPO1-153 Double or Triple Variants

Further, double or triple variants in which two or three amino acids were substituted were constructed using the same primers as used for single variants. The double variants were designed to have substitutions at two positions both of which induce an increase in protease resistance or one of which induce an increase in protease resistance while the other induces a decrease in protease resistance. As for triple variants, they were constructed from the double variants of increased protease resistance by substitution at one additional position which induces an increase in protease resistance. A design list for double or triple variants is given in Table 8, below.

**TABLE 8 Design List for Double and Triple Variants**

| Variant No. | Mutation | Variant No. | Mutation |
|---|---|---|---|
| TPO1-153-201 (53/133)* | K52N/V139I | TPO1-153-215 (73/132) | L71I/K138T |
| TPO1-153-202 (11/132) | L12I/K138T | TPO1-153-216 (79/132) | V74T/K138T |
| TPO1-153-203 (16/132) | R17Q/K138T | TPO1-153-217 (81/132) | A76S/K138T |
| TPO1-153-204 (20/132) | H20Q/K138T | TPO1-153-218 (87/132) | L81I/K138T |
| TPO1-153-205 (22/132) | V21T/K138T | TPO1-153-219 (97/132) | L99I/K138T |
| TPO1-153-206 (37/132) | L40I/K138T | TPO1-153-220 (112/132) | H121N/K138T |
| TPO1-153-207 (39/132) | A43S/K138T | TPO1-153-221 (115/132) | K122Q/K138T |
| TPO1-153-208 (42/132) | V44T/K138T | TPO1-153-222 (117/132) | D123N/K138T |
| TPO1-153-209 (53/132) | K52N/K138T | TPO1-153-223 (122/132) | H133N/K138T |
| TPO1-153-210 (57/132) | E57Q/K138T | TPO1-153-301 (7/57/132) | L9I/E57Q/K138T |
| TPO1-153-211 (60/132) | K59N/K138T | TPO1-153-302 (22/57132) | V21T/E57Q/K138T |
| TPO1-153-212 (66/132) | G65S/K138T | TPO1-153-224 (16/53) | R17Q/K52N |
| TPO1-153-213 (67/132) | A66S/K138T | TPO1-153-225 (30/53) | V32I/K52N |
| TPO1-153-214 (70/132) | V67T/K138T | | |

| | | | |
|---|---|---|---|
| *("X/Y" represents double mutations as in TPO1-153-X and TPO1-153-Y, and "X/Y/Z" represents triple mutations as in TPO1-153-X, TPO1-153-Y, and TPO1-153-Z (wherein X, Y and Z are constant numbers) | | | |

### [EXAMPLE 14] Construction of TPO1-153 Double or Triple Variants

The double or triple variants listed in Table 8 were constructed from the single variants using the primers of Table 3. For the double variants, mutation was induced at an additional one position in the single variants. As for the triple variants, they resulted from mutation at one additional position in addition to the double variants.

TPO1-153 double variants were prepared in the same manner as in Example 8, with the exception that primers of Table 3 were used in combination as indicated in Table 8 while the single variant pcDNA3.3-GH-TPO1-153-132 plasmid was used as a template instead of the pcDNA3.3-GH_TPO1-153 plasmid.

Thereafter, the PCR solutions were treated for 5 min with DpnI to degrade the DNA of E. coli. The PCR products thus obtained were introduced into E. coli XL1-blue cells. The recombinant plasmids isolated from the transformants were subjected to base sequencing to confirm the site-directed mutagenesis.

The triple variants were prepared from the base-sequenced double variant plasmid as a template in the same manner as described above. Base sequencing confirmed the completion of site-directed mutagenesis.

### [EXAMPLE 15] Expression and Quantification of TPO1-153 Double or Triple Variants

After being isolated, the variants plasmids constructed in Example 14 were expressed in HEK293 cells and quantified in the same manner as in Examples 9 and 10.

Expression levels of TPO1-153 and TPO1-153 variants determined by ELISA are given in Table 9, below.

**TABLE 9 Expression Levels of TPO1-153 Double and Triple Variants**

| Variant No. | Expression Level (ng/ml) | Variant No. | Expression Level (ng/ml) |
|---|---|---|---|
| TPO1-153-201 (53/133) | 1045 | TPO1-153-215 (73/132) | 357 |
| TPO1-153-202 (11/132) | 1142 | TPO1-153-216 (79/132) | 1649 |
| TPO1-153-203 (16/132) | 732 | TPO1-153-217 (81/132) | 2250 |
| TPO1-153-204 (20/132) | 1537 | TPO1-153-218 (87/132) | 308 |
| TPO1-153-205 (22/132) | 1567 | TPO1-153-219 (97/132) | 1114 |
| TPO1-153-206 (37/132) | 881 | TPO1-153-220 (112/132) | 890 |
| TPO1-153-207 (39/132) | 1761 | TPO1-153-221 (115/132) | 565 |
| TPO1-153-208 (42/132) | 1096 | TPO1-153-222 (117/132) | 254 |
| TPO1-153-209 (53/132) | 1658 | TPO1-153-223 (122/132) | 916 |
| TPO1-153-210 (57/132) | 967 | TPO1-153-301 (7/57/132) | 525 |
| TPO1-153-211 (60/132) | 65 | TPO1-153-302 (22/57/132) | 968 |
| TPO1-153-212 (66/132) | 880 | TPO1-153-224 (16/53) | 131 |
| TPO1-153-213 (67/132) | 1379 | TPO1-153-225 (30/53) | 294 |
| TPO1-153-214 (70/132) | 2392 | | |

### [EXAMPLE 16] In vitro Assay of TPO1-153 and TPO1-153 Double or Triple Variants for Biological Activity in Terms of STAT5 Phosphorylation

In vitro biological activity was assayed in the same manner as in Example 11, with the exception that TPO1-153 double or triple variants were used instead of TPO1-153 variants.

The biological activities of the double or triple TPO1-153 variants in terms of STAT5 phosphorylation are given in Table 10, below.

**TABLE 10 Biological Activity of TPO1-153 Double or Triple Variants**

| Variant NO. | Relative Activity | Variant NO. | Relative Activity |
|---|---|---|---|
| TPO1-153-201 (53/133) | 36% | TPO1-153-215 (73/132) | -4% |
| TPO1-153-202 (11/132) | 2% | TPO1-153-216 (79/132) | -6% |
| TPO1-153-203 (16/132) | -1% | TPO1-153-217 (81/132) | -3% |
| TPO1-153-204 (20/132) | -1% | TPO1-153-218 (87/132) | 0% |
| TPO1-153-205 (22/132) | 1% | TPO1-153-219 (97/132) | 3% |
| TPO1-153-206 (37/132) | -1% | TPO1-153-220 (112/132) | 2% |
| TPO1-153-207 (39/132) | -2% | TPO1-153-221 (115/132) | 0% |
| TPO1-153-208 (42/132) | -7% | TPO1-153-222 (117/132) | 2% |
| TPO1-153-209 (53/132) | -6% | TPO1-153-223 (122/132) | -2% |
| TPO1-153-210 (57/132) | -3% | TPO1-153-301 (7/57/132) | -5% |
| TPO1-153-211 (60/132) | 5% | TPO1-153-302 (22/57/132) | -1% |
| TPO1-153-212 (66/132) | -1% | TPO1-153-224 (16/53) | 34% |
| TPO1-153-213 (67/132) | -2% | TPO1-153-225 (30/53) | 105% |
| TPO1-153-214 (70/132) | -1% | | |

### [EXAMPLE 17] Resistance of TPO1-153 Double or Triple Variants to Proteases

Resistance of the TPO1-153 double or triple variants to a mix of 10 different proteases was determined at 25°C in terms of half life in the same manner as in Example 12, with the exception that the TPO1-153 double or triple variants were used instead of the TPO1-153 single variants.

Protease resistance of the TPO1-153 double or triple variants is given in Table 11, below.

**TABLE 11 Protease Resistance of TPO1-153 Double or Triple Variants**

| Variant No. | Resistance Relative to TPO1-153 | Variant No. | Resistance Relative to TPO1-153 |
|---|---|---|---|
| TPO1-153-201 (53/133) | 489% | TPO1-153-215 (73/132) | 380% |
| TPO1-153-202 (11/132) | 217% | TPO1-153-216 (79/132) | 317% |
| TPO1-153-203 (16/132) | 531% | TPO1-153-217 (81/132) | 400% |
| TPO1-153-204 (20/132) | 811% | TPO1-153-218 (87/132) | 506% |
| TPO1-153-205 (22/132) | 342% | TPO1-153-219 (97/132) | 248% |
| TPO1-153-206 (37/132) | 335% | TPO1-153-220 (112/132) | 621% |
| TPO1-153-207 (39/132) | 347% | TPO1-153-221 (115/132) | n/a |
| TPO1-153-208 (42/132) | 1006% | TPO1-153-222 (117/132) | 232% |
| TPO1-153-209 (53/132) | 856% | TPO1-153-223 (122/132) | 4175% |
| TPO1-153-210 (57/132) | 616% | TPO1-153-301 (7/57/132) | 315% |
| TPO1-153-211 (60/132) | 733% | TPO1-153-302 (22/57/132) | 1005% |
| TPO1-153-212 (66/132) | 306% | TPO1-153-224 (16/53) | 171% |
| TPO1-153-213 (67/132) | 982% | TPO1-153-225 (30/53) | 154% |
| TPO1-153-214 (70/132) | 220% | | |

| | | | |
|---|---|---|---|
| *n/a: not applicable | | | |

[EXAMPLE 18] Amplification of TP07-151 Gene and Construction of Expression Vector for TP07-151 Gene

A gene coding for TP07-151 was amplified by PCR while pcDNA3.3-TPO1-153, prepared in Example 2, served as a template. Primers for use in the construction of GH-TP07-151 were TPO1-153-N7-hGHN1 (ttcaagagggcagtgcctgtgacctccgagtcctcagtaaactgcttc), TPOI-153hGH-N2 (tctgcctgccctggcttcaagagggcagtgccagcc), TPO1-153hGH-N3 (tggcttttggcctgctctgcctgccctggcttcaag), TPO1-153hGH-N4 (ggacgtccctgctcctggcttttggcctgctctgcc), TPO1-153hGH-N5 (accatggctacaggctcccggacgtccctgctcctggct) and TPO151-C (ttagcagagggtggaccctc). Serial PCR reactions deleted codons for amino acids at positions 152, 153, and 1 to 6. The resulting gene encodes a deletion TPO variant improved in ability to be secreted and was cloned into pcDNA3.3-TOPO TA to construct a recombinant vector, named pcDNA3.3-GH_TPO7-151. Base sequencing confirmed the cloning of a correct GH-TP07-151 gene.

In brief, while pcDNA3.3-TPO1-153 was used as a template, PCR with a serial of primers from TPO-N7-hGH-N1 to N5 gave a final PCR product having a signal sequence of hGH. For this PCR, a PCR solution containing 5 µL of 50 ng cDNA, 3 µL of 10mM N-primer, 3 µL of 10mM C-primer, 50 µL of Accupower PCR premix, and 39 µL of H₂O was used. PCR started with denaturing at 95°C for 10 min and was performed with 25 cycles of denaturing at 95°C for 1 min, annealing at 55°C for 1 min and extension at 72°C for 1 min, followed by extension at 72°C for 5 min. The PCR product thus obtained was cloned into pcDNA3.3-TOPO TA in the presence of ligase and introduced into E. coli XL1-blue.

Base sequencing confirmed the cloning of a correct TP07-151 gene into the vector.

### [EXAMPLE 19] Expression and Quantification of TP07-151

TP07-151 was expressed in HEK293 cells and quantified in the same manner as in Examples 9 and 10, with the exception that pcDNA3.3-GH_TPO7-151, constructed in Example 18, was used instead of pcDNA3.3-GH-TPO1-153.

TP07-151 was found to be expressed in an amount of 60 ng/mL as measured by ELISA.

### [EXAMPLE 20] In vitro Assay of TP07-151 for Biological Activity in Terms of STAT5 Phosphorylation

In vitro biological activity was assayed in the same manner as in Example 11, with the exception that TPO1-153 and TP07-151 were used instead of TPO1-153 variants.

The biological activity of TP07-151 in terms of STAT5 phosphorylation was found to be about 20% of that of TPO1-153.

### [EXAMPLE 21] Design and Construction of TP07-151 Single Variants

The design and construction of TP07-151 variants was achieved in the same manner as in Examples 7 and 8. The designed TP07-151 single variants are listed in Table 12, below. The mutation positions and the substitutive amino acid candidates were determined as seen in Table 2.

Site-specific TP1-153 single variants were constructed using the primers of Table 3 which were designed to correspond to the mutation of each variant in the same manner as in Example 8, with the exception that pcDNA3.3-GH_TP07-151 was used as a template instead of pcDNA3.3-GH TPO1-153. The clones thus obtained were inserted into suitable vectors and subjected to base sequencing to confirm correct site-specific mutations.

**TABLE 12 Designed TP07-151 Single Variants**

| Variant No. | Mutation | Variant No. | Mutation |
|---|---|---|---|
| TP07-151-1001 | E56N | TP07-151-1021 | M143N |
| TP07-151-1002 | K138T | TP07-151-1022 | R78Q |
| TP07-151-1003 | K138Q | TP07-151-1023 | V21I |
| TP07-151-1004 | V145T | TP07-151-1024 | A103T |
| TP07-151-1005 | G137S | TP07-151-1025 | K59N |
| TP07-151-1006 | V74I | TP07-151-1026 | R136Q |
| TP07-151-1007 | K52N | TP07-151-1027 | V32I |
| TP07-151-1008 | L107I | TP07-151-1028 | G73S |
| TP07-151-1009 | V139I | TP07-151-1029 | H33Q |
| TP07-151-1010 | F141S | TP07-151-1030 | L16I |
| TP07-151-1011 | G49S | TP07-151-1031 | K59Q |
| TP07-151-1012 | V44T | TP07-151-1032 | V67T |
| TP07-151-1013 | D45N | TP07-151-1033 | V97I |
| TP07-151-1014 | R25Q | TP07-151-1034 | K122N |
| TP07-151-1015 | R98Q | TP07-151-1035 | H33N |
| TP07-151-1016 | A103S | TP07-151-1036 | L22I |
| TP07-151-1017 | H133N | TP07-151-1037 | F46I |
| TP07-151-1018 | L99I | TP07-151-1038 | R140Q |
| TP07-151-1019 | G79S | TP07-151-1039 | L112I |
| TP07-151-1020 | H133Q | TP07-151-1040 | H23Q |

**TABLE 13 Primers for Site-Directed Mutagenesis of TP07-151**

| Variant No. | mutation | Primer Direction | Primer sequence |
|---|---|---|---|
| TP07-151-1001 | E56N | Forward | 5'-gaatggaaaacccagatgAATgagaccaaggcac-3' |
| | | Reverse | 5'-gtgccttggtctcATTcatctgggttttccattc-3' |
| TP07-151-1002 | K138T | Forward | 5'-cacctgctccgaggaACCgtgcgtttcc-3' |
| | | Reverse | 5'-ggaaacgcacGGTtcctcggagcaggtg-3' |
| TP07-151-1003 | K138Q | Forward | 5'-cacctgctccgaggaCAGgtgcgtttcc-3' |
| | | Reverse | 5'-ggaaacgcacCTGtcctcggagcaggtg-3' |
| TP07-151-1004 | V145T | Forward | 5'-gtgcgtttcctgatgcttACCggagggtccaccc-3' |
| | | Reverse | 5'-gggtggaccctccGGTaagcatcaggaaacgcac-3' |
| TP07-151-1005 | G137S | Forward | 5'-caacacctgctccgaAGCaaggtgcgtttcctga-3' |
| | | Reverse | 5'-tcaggaaacgcaccttGCTtcggagcaggtgttg-3' |
| TP07-151-1006 | V74I | Forward | 5'-cttctgctggagggaATTatggcagcacgggga-3' |
| | | Reverse | 5'-tccccgtgctgccatAATtccctccagcagaag-3' |
| TPO7-151-1007 | K52N | Forward | 5'-gactttagcttgggagaatggAATacccagatggag- 3' |
| | | Reverse | 5'-ctccatctgggtATTccattctcccaagctaaagtc- 3' |
| TP07-151-1008 | L107I | Forward | 5'-ggccctgcagagcATCcttggaaccca-3' |
| | | Reverse | 5'-tgggttccaagGATgctctgcagggcc-3' |
| TP07-151-1009 | V139I | Forward | 5'-ctgctccgaggaaagATCcgtttcctgatgcttg-3' |
| | | Reverse | 5'-caagcatcaggaaacgGATctttcctcggagcag-3' |
| TP07-151-1010 | F141S | Forward | 5'-gctccgaggaaaggtgcgtAGCctgatgct-3' |
| | | Reverse | 5'-agcatcagGCTacgcacctttcctcggagc-3' |
| TP07-151-1011 | G49S | Forward | 5'-ctgtggactttagcttgAGCgaatggaaaaccca-3' |
| | | Reverse | 5'-tgggttttccattcGCTcaagctaaagtccacag-3' |
| TP07-151-1012 | V44T | Forward | 5'-ctgtcctgctgcctgctACCgactttagcttggg-3' |
| | | Reverse | 5'-cccaagctaaagtcGGTagcaggcagcaggacag-3' |
| TP07-151-1013 | D45N | Forward | 5'-ctgctgcctgctgtgAATtttagcttgggag-3' |
| | | Reverse | 5'-ctcccaagctaaaATTcacagcaggcagcag-3' |
| TP07-151-1014 | R25Q | Forward | 5'-catgtccttcacagcCAGctgagccagtgcccag-3' |
| | | Reverse | 5'-ctgggcactggctcagCTGgctgtgaaggacatg-3' |
| TP07-151-1015 | R98Q | Forward | 5'-tttctggacaggtcCAGctcctccttggggcc-3' |
| | | Reverse | 5'-ggccccaaggaggagCTGgacctgtccagaaa-3' |
| TP07-151-1016 | A103S | Forward | 5'-gtctcctccttgggAGCctgcagagcctcc-3' |
| | | Reverse | 5'-ggaggctctgcagGCTcccaaggaggagac-3' |
| TP07-151-1017 | H133N | Forward | 5'-gccatcttcctgagcttccaaAATctgctccga-3' |
| | | Reverse | 5'-tcggagcagATTttggaagctcaggaagatggc-3' |
| TP07-151-1018 | L99I | Forward | 5'-ctttctggacaggtccgtATTctccttggg-3' |
| | | Reverse | 5'-cccaaggagAATacggacctgtccagaaag-3' |
| TP07-151-1019 | G79S | Forward | 5'-tgatggcagcacggAGCcaactgggacccac-3' |
| | | Reverse | 5'-gtgggtcccagttgGCTccgtgctgccatca-3' |
| TP07-151-1020 | H133Q | Forward | 5'-cctgagcttccaaCAGctgctccgaggaaag-3' |
| | | Reverse | 5'-ctttcctcggagcagCTGttggaagctcagg-3' |
| TP07-151-1021 | M143N | Forward | 5'-ggtgcgtttcctgAATcttgtaggagggtccacc-3' |
| | | Reverse | 5'-ggtggaccctcctacaagATTcaggaaacgcacc-3' |
| TP07-151-1022 | R78Q | Forward | 5'-agtgatggcagcaCAGggacaactgggac-3' |
| | | Reverse | 5'-gtcccagttgtccCTGtgctgccatcact-3' |
| TP07-151-1023 | V21I | Forward | 5'-ctgcttcgtgactcccatATCcttcacagcaga-3' |
| | | Reverse | 5'-tctgctgtgaagGATatgggagtcacgaagcag-3' |
| TP07-151-1024 | A103T | Forward | 5'-ctcctccttgggACCctgcagagcc-3' |
| | | Reverse | 5'-ggctctgcagGGAcccaaggaggag-3' |
| TP07-151-1025 | K59N | Forward | 5'-cagatggaggagaccAATgcacaggacattctg-3' |
| | | Reverse | 5'-cagaatgtcctgtgcATTggtctcctccatctg-3' |
| TP07-151-1026 | R136Q | Forward | 5'-ttccaacacctgctcCAGggaaaggtgcgtttc-3' |
| | | Reverse | 5'-gaaacgcacctttccCTGgagcaggtgttggaa-3' |
| TP07-151-1027 | V32I | Forward | 5'-gccagtgcccagagATTcaccctttgcct-3' |
| | | Reverse | 5'-aggcaaagggtgAATctctgggcactggc-3' |
| TP07-151-1028 | G73S | Forward | 5'-cccttctgctggagAGCgtgatggcagcacg-3' |
| | | Reverse | 5'-cgtgctgccatcacGCTctccagcagaaggg-3' |
| TP07-151-1029 | H33Q | Forward | 5'-ccagtgcccagaggttCAGcctttgccta-3' |
| | | Reverse | 5'-taggcaaaggCTGaacctctgggcactgg-3' |
| TP07-151-1030 | L16I | Forward | 5'-agtcctcagtaaactgATCcgtgactcccatgtc-3' |
| | | Reverse | 5'-gacatgggagtcacgGATcagtttactgaggact-3' |
| TP07-151-1031 | K59Q | Forward | 5'-cagatggaggagaccCAGgcacaggacattc-3' |
| | | Reverse | 5'-gaatgtcctgtgcCTGggtctcctccatctg-3' |
| TP07-151-1032 | V67T | Forward | 5'-ggacattctgggagcaACCacccttctgctggag-3' |
| | | Reverse | 5'-ctccagcagaagggtGGTtgctcccagaatgtcc-3' |
| TP07-151-1033 | V97I | Forward | 5'-cagctttctggacagATTcgtctcctccttg-3' |
| | | Reverse | 5'-caaggaggagacgAATctgtccagaaagctg-3' |
| TP07-151-1034 | K122N | Forward | 5'-ggaccacagctcacAATgatcccaatgccatct-3' |
| | | Reverse | 5'-agatggcattgggatcATTgtgagctgtggtcc-3' |
| TP07-151-1035 | H33N | Forward | 5'-agccagtgcccagaggttAATcctttgcc-3' |
| | | Reverse | 5'-ggcaaaggATTaacctctgggcactggct-3' |
| TP07-151-1036 | L22I | Forward | 5'-tcgtgactcccatgtcATTcacagcagactgag-3' |
| | | Reverse | 5'-ctcagtctgctgtgAATgacatgggagtcacga-3' |
| TP07-151-1037 | F46I | Forward | 5'-gctgcctgctgtggacATTagcttgggagaatg-3' |
| | | Reverse | 5'-cattctcccaagctAATgtccacagcaggcagc-3' |
| TP07-151-1038 | R140Q | Forward | 5'-ctccgaggaaaggtgCAGttcctgatgcttgtag-3' |
| | | Reverse | 5'-ctacaagcatcaggaaCTGcacctttcctcggag-3' |
| TP07-151-1039 | L112I | Forward | 5'-tccttggaacccagATTcctccacagggc-3' |
| | | Reverse | 5'-gccctgtggaggAATctgggttccaagga-3' |
| TP07-151-1040 | H23Q | Forward | 5'-cgtgactcccatgtccttCAGagcagactgag-3' |
| | | Reverse | 5'-ctcagtctgctCTGaaggacatgggagtcacg-3' |

### [EXAMPLE 22] Expression and Quantification of TP07-151 Single Variants

The TP07-151 single variants constructed in Example 21 were expressed in HEK293 cells and quantified in the same manner as in Examples 9 and 10.

Expression levels of TP07-151 single variants are given in Table 14, below.

**TABLE 14 Expression Levels of TP07-151 Variants**

| Variant No. | Expression Level(ng/ml) | Variant No. | Expression Level (ng/ml) |
|---|---|---|---|
| TPO7-151-1001 | 137 | TPO7-151-1021 | 61 |
| TPO7-151-1002 | 256 | TPO7-151-1022 | 26 |
| TP07-151-1003 | 28 | TP07-151-1023 | 22 |
| TP07-151-1004 | 206 | TP07-151-1024 | 51 |
| TP07-151-1005 | 44 | TP07-151-1025 | 32 |
| TPO7-151-1006 | 71 | TPO-151-1026 | 32 |
| TP07-151-1007 | 38 | TP07-151-1027 | 17 |
| TP07-151-1008 | 43 | TP07-151-1028 | 37 |
| TP07-151-1009 | 39 | TP07-151-1029 | 23 |
| TP07-151-1010 | 203 | TP07-151-1030 | 37 |
| TP07-151-1011 | 28 | TP07-151-1031 | 28 |
| TP07-151-1012 | 39 | TP07-151-1032 | 43 |
| TP07-151-1013 | 72 | TP07-151-1033 | 34 |
| TP07-151-1014 | 29 | TP07-151-1034 | 22 |
| TP07-151-1015 | 19 | TP07-151-1035 | 25 |
| TP07-151-1016 | 22 | TP07-151-1036 | 6 |
| TP07-151-1017 | 22 | TP07-151-1037 | 23 |
| TP07-151-1018 | 41 | TP07-151-1038 | 11 |
| TP07-151-1019 | 74 | TP07-151-1039 | 38 |
| TP07-151-1020 | 20 | TP07-151-1040 | 17 |

### [EXAMPLE 23] In vitro Assay of TP07-151 Single Variants for Biological Activity in Terms of STAT5 Phosphorylation

In vitro biological activity was assayed in the same manner as in Example 11, with the exception that single TP07-151 variants were used instead of single TPO1-153 variants.

The biological activities of the single TP07-151 variants in terms of STAT5 phosphorylation in M-o7e cells were measured and are given in Table 15, below.

**TABLE 15 Biological Activity of TP07-151 Single Variants**

| Variant No. | Activity Relative to TP07-151 | Variant No. | Activity Relative to TP07-151 |
|---|---|---|---|
| TP07-151-1001 | 46% | TP07-151-1021 | -4% |
| TP07-151-1002 | -5% | TP07-151-1022 | 38% |
| TP07-151-1003 | 1% | TP07-151-1023 | 185% |
| TP07-151-1004 | 170% | TP07-151-1024 | 119% |
| TP07-151-1005 | -5% | TP07-151-1025 | -7% |
| TP07-151-1006 | 54% | TP07-151-1026 | 15% |
| TP07-151-1007 | 153% | TP07-151-1027 | 141% |
| TP07-151-1008 | 146% | TP07-151-1028 | 121% |
| TP07-151-1009 | 21% | TP07-151-1029 | 132% |
| TP07-151-1010 | -11% | TP07-151-1030 | 115% |
| TP07-151-1011 | 202% | TP07-151-1031 | 1% |
| TP07-151-1012 | 33% | TP07-151-1032 | 143% |
| TP07-151-1013 | -5% | TP07-151-1033 | 89% |
| TP07-151-1014 | 47% | TP07-151-1034 | 145% |
| TP07-151-1015 | -13% | TP07-151-1035 | 144% |
| TP07-151-1016 | 134% | TP07-151-1036 | 124% |
| TP07-151-1017 | 75% | TP07-151-1037 | -19% |
| TP07-151-1018 | 156% | TP07-151-1038 | -24% |
| TP07-151-1019 | 156% | TP07-151-1039 | 138% |
| TP07-151-1020 | 105% | TP07-151-1040 | 144% |

### [EXAMPLE 24] Resistance of TP07-151 Single Variants to Proteases

Resistance of the TP07-151 single variants to a mix of 10 different proteases was determined in terms of half life in the same manner as in Example 12, with the exception that the single TP07-151 variants were used instead of the single TPO 153 variants. The results are given in Table 16, below.

**TABLE 16 Protease Resistance of TP07-151 Variants**

| Variant No. | Resistance Relative to TP07-151 | Variant No. | Resistance Relative to TP07-151 |
|---|---|---|---|
| TP07-151-1001 | 364% | TP07-151-1021 | 88% |
| TP07-151-1002 | 13475% | TP07-151-1022 | 321% |
| TP07-151-1003 | 280% | TP07-151-1023 | 572% |
| TP07-151-1004 | 442% | TP07-151-1024 | 168% |
| TP07-151-1005 | 136% | TP07-151-1025 | 567% |
| TP07-151-1006 | 212% | TP07-151-1026 | 712% |
| TP07-151-1007 | 175% | TP07-151-1027 | 131% |
| TP07-151-1008 | 196% | TP07-151-1028 | 439% |
| TP07-151-1009 | 432% | TP07-151-1029 | 368% |
| TP07-151-1010 | 393% | TP07-151-1030 | 199% |
| TP07-151-1011 | 329% | TP07-151-1031 | 313% |
| TP07-151-1012 | 127% | TP07-151-1032 | 364% |
| TP07-151-1013 | 291% | TP07-151-1033 | 222% |
| TP07-151-1014 | 456% | TP07-151-1034 | 907% |
| TP07-151-1015 | 134% | TP07-151-1035 | 230% |
| TP07-151-1016 | 396% | TP07-151-1036 | 95% |
| TP07-151-1017 | 539% | TP07-151-1037 | 358% |
| TP07-151-1018 | 141% | TP07-151-1038 | 161% |
| TP07-151-1019 | 382% | TP07-151-1039 | 216% |
| TP07-151-1020 | 120% | TP07-151-1040 | 83% |

### [EXAMPLE 25] Construction of TP07-151 Double and Triple Variants

As described in Example 13, TP07-151 double variants were constructed by introducing mutations at amino acid positions corresponding to those of the TPO153 double variants listed in Table 8. The resulting variants are listed in Table 17, below. For TP07-151 double variants, the TP07-151 variants were allowed to undergo mutation at one additional position using primers corresponding to the mutation given in Table 17. As for TP07-151 triple variants, they were constructed from the double variants by inducing mutation at one additional desired position.

**TABLE 17 Design List of Double and Triple Variants**

| Variant No. | mutation |
|---|---|
| TP07-151-1201 | A76S/ K138T |
| TP07-151-1202 | K52N/ K138T |
| TP07-151-1203 | V21T/ K138T |
| TP07-151-1204 | H20Q / K38T |
| TP07-151-1205 | A66S/ K38T |
| TP07-151-1206 | L12I/ K38T |
| TP07-151-1207 | L99I/ K138T |
| TP07-151-1208 | K52N/V139I |
| TP07-151-1209 | V32I/ K52N |
| TP07-151-1210 | R17Q/K52N |

TP07-151 double variants were prepared using in the same manner as in Example 14, with the exception that the primers of Table 3 were used in combination as indicated in Table 17 while the single variant pcDNA3.3-GH_TPO7-151-132 plasmid was used as a template, instead of the pcDNA3.3-GH-TPO1-153-132 plasmid.

Thereafter, the PCR solutions were treated for 5 min with DpnI to degrade the DNA of E. coli. The PCR products thus obtained were introduced into E. coli XLI-blue cells. The recombinant plasmids isolated from the transformants were subjected to base sequencing to confirm the site-directed mutagenesis.

The triple variants were prepared from the base-sequenced double variant plasmid in the same manner as described above.

### [EXAMPLE 26] Expression and Quantification of TP07-151 Double or Triple Variants

After being isolated, the variants plasmids constructed in Example 25 were expressed in HEK293 cells and quantified in the same manner as in Examples 9 and 10.

The TP07-151 double variants were found to have an expression level of from 200 to 700 ng/ml as measured by ELISA.

### [EXAMPLE 27] In vitro Assay of TP07-151 Double or Triple Variants for Biological Activity in Terms of STAT5 Phosphorylation

In vitro biological activity was assayed in the same manner as in Example 11, with the exception that the TP07-151 double or triple variants were used instead of the TPO1-153 variants.

The biological activities of the TP07-151 double or triple variants in terms of STAT5 phosphorylation in comparison with those of TP07-151 are given in Table 18, below.

**TABLE 18 Relative Biological Activity of TP07-151 Double or Triple Variants**

| Variant No. | Activity Relative to TP07-151 |
|---|---|
| TP07-151-1201 | -2% |
| TP07-151-1202 | -5% |
| TP07-151-1203 | 2% |
| TP07-151-1204 | -1% |
| TP07-151-1205 | 1% |
| TP07-151-1206 | 0% |
| TP07-151-1207 | -3% |
| TP07-151-1208 | 40% |
| TP07-151-1209 | 95% |
| TP07-151-1210 | 25% |

### [EXAMPLE 28] Resistance of TP07-151 Double or Triple Variants to Proteases

Resistance of the TP07-151 double or triple variants to a mix of 10 different proteases of Example 12 was determined in terms of half life in the same manner as in Example 12, with the exception that the TP07-151 double or triple variants were used instead of the TPO1-153 single variants.

Protease resistance of the TP07-151 double or triple variants relative to the TP07-151 was measured and the results are given in Table 19, below.

**TABLE 19 Protease Resistance of Double and Triple TP07-151 Variants**

| Variant No. | Resistance Relative to TP07-151 |
|---|---|
| TP07-151-1201 | 48111% |
| TP07-151-1202 | 144333% |
| TP07-151-1203 | n/a |
| TP07-151-1204 | 5413% |
| TP07-151-1205 | 1968% |
| TP07-151-1206 | 1493% |
| TP07-151-1207 | 2547% |
| TP07-151-1208 | n/a |
| TP07-151-1209 | 401% |
| TP07-151-1210 | 1102% |

| | |
|---|---|
| * n/a: not applicable | |

## Claims

1. A human thrombopoietin (TPO) fragment variant, having a modified amino acid sequence, wherein the modified amino acid sequence comprises one or more amino acid substitution(s) at position(s) selected from amongst positions 3, 6, 8 to 12, 14 to 18, 20 to 23, 25, 26, 31 to 34, 39 to 41, 43 to 46, 48 to 52, 55 to 57, 59, 60, 62, 64 to 67, 69 to 79, 81, 86, 89 to 91, 93, 95, 97 to 104, 107 to 109, 112, 116, 117, 120 to 123, 126, 128, 129, 131, 133 to 147, 150, and 152 in the amino acid sequence of the native TPO fragment 1-153 (TPO1-153) represented by SEQ ID NO: 1.

2. The TPO fragment variant of claim 1, wherein the one or more amino acid substitution(s) is(are) at position(s) selected from amongst positions 3, 9, 14 , 16 to 18, 20 to 23, 25, 32, 40, 43 to 46, 51, 52, 56, 57, 59, 62, 65 to 67, 69, 73 to 76, 79, 81, 99, 103, 104, 109, 117, 122, 129, 133, 135 to 139, 140 to 145, 150, and 152 in the amino acid sequence of TPO1-153 represented by SEQ ID NO: 1.

3. The TPO fragment variant of claim 1, wherein the one or more amino acid substitution(s) is(are) at position(s) selected from amongst positions 17, 20, 21, 32, 52, 59, 66, 67, 138, 139, 140, 141, 142, 145, 150, and 152 in the amino acid sequence of TPO1-153 represented by SEQ ID NO: 1.

4. The TPO fragment variant of claim 1, wherein the amino acid residues at positions 1 to 6, 152 and 153 of the amino acid sequence of TP01-153 represented by SEQ ID NO: 1 are deleted.

5. The TPO fragment variant of claim 4, wherein the one or more amino acid substitution(s) is(are) at positions 9, 14, 16 to 18, 20 to 23, 25, 32, 33, 40, 43 to 46, 49, 51, 52, 56, 57, 59, 62, 65 to 67, 69, 73 to 76, 78, 79, 81, 97, 99, 103 , 104, 107, 109, 112, 117, 122, 129, 133, and 135 to 145 in the TP01-153 in the amino acid sequence of TPO1-153 represented by SEQ ID NO: 1.

6. The TPO fragment variant of claim 5, wherein the one or more amino acid substitution(s) is(are) at positions 16, 21, 23, 25, 32, 33, 44 to 46, 49, 52, 56, 59, 67, 73, 74, 78, 79, 97, 98, 99, 103, 107, 112, 133, 136 to 141, and 145 in the amino acid sequence of TPO1-153 represented by SEQ ID NO: 1.

7. The TPO fragment variant of any one of claims 1 to 6, wherein the one or more amino acid substitution(s) is(are) selected from amongst amino acid substitutions of A with S or T; D with N or Q; L with I; R with Q or N; V with T or I; K with N, Q or T; H with Q or N; E with Q, N, or S; P with S; F with I or S; G with S; W with I or S; and M with I or N.

8. The TPO fragment variant of claim 1, wherein the one or more amino acid substitution(s) is(are) selected from amongst substitutions of A3S, A3T, L9I, K14N, K14Q, L16I, R17Q, D18Q, H20Q, V21I, V21T, L22I, H23N, R25Q, R25N, V32I, L40I, A43S, V44I, V44T, D45N, F46I, W51S, K52Q, K52N, E56N, E57N, K59N, D62Q, G65S, A66S, V67T, L69I, G73S, V74I, V74T, M75I, A76S, G79S, L81I, L99I, A103T, L104I, G109S, R117Q, K122Q, L129I, H133Q, L135I, R136Q, G137S, K138N, K138Q, K138S, K138T, V139I, V139T, R140Q, F141I, F141S, L142I, M143I, M143N, L144I, V145T, L150I, and V152T.

9. The TPO fragment variant of claim 1, wherein the one or more amino acid substitution(s) is(are) selected from amongst substitutions of R17Q, H20Q, V21T, V32I, K52N, K59N, A66S, V67T, K138Q, K138S, K138T, V139I, V139T, F141I, F141S, and L142I.

10. The TPO fragment variant of claim 4, wherein the one or more amino acid substitution(s) is(are) selected from amongst substitutions of L6I, L9I, K14N, K14Q, L16I, R17Q, D18Q, H20Q, V21I, V21T, L22I, H23N, H23Q, R25Q, R25N, V32I, H33N, H33Q, L40I, A43S, V44I, V44T, D45N, F46I, G49S, W51S, K52Q, K52N, E56N, E57N, K59N, K59Q, D62Q, G65S, A66S, V67T, L69I, G73S, V74I, V74T, M75I, A76S, R78Q, G79S, L81I, V97I, R98Q, L99I, A103S, A103T, L104I, L107I, G109S, L112I, R117Q, K122Q, L129I, H133N, H133Q, L135I, R136Q, G137S, K138N, K138Q, K138T, V139I, V139T, R140Q, F141I, F141S, L142I, M143I, M143N, L144I, and V145T.

11. The TPO fragment variant of claim 4, wherein the one or more amino acid substitution(s) is(are) selected from amongst substitutions of L16I, V21I, V32I, H33N, H33Q, G49S, K52N, V67T, G73S, G79S, L99I, A103S, A103T, L107I, L112I, H133Q, and V145T.

12. The TPO fragment variant of claim 1, wherein the modified amino acid sequence comprises 2 amino acid substitutions and the 2 amino acid substitutions are one selected from amongst replacements of V32I/K52N, K52N/K138S, and K52N/V139I.

13. The TPO fragment variant of claim 4, wherein the modified amino acid sequence comprises 2 amino acid substitutions and the 2 amino acid substitutions are one selected from amongst replacements of V32I/K52N, K52N/K138S, and K52N/V139I.

14. A gene, having a nucleotide sequence encoding the modified amino acid sequence of any one of claims 1 to 13.

15. A vector, carrying the gene of claim 14.

16. The vector of claim 15, carrying a TPO gene as represented by the cleavage map of FIG. 2B.

17. A microbial or animal cell, transformed with the vector of claim 15 or Claim 16.

18. The microbial or animal cell of claim 17, that is E.coli BL21(DE3) (Accession No: KCTC11453BP), CHO cell, COS-7 cell or HEK293 cell.

19. A pharmaceutical preparation, comprising the TPO fragment variant of any one of claims 1 to 13.

20. The pharmaceutical preparation of claim 19, further comprising a pharmaceutically acceptable excipient.

21. The pharmaceutical preparation of Claim 19, that is in a form of a formulation selected from the group consisting of an oral formulation, an inhaler, an injection, a transmucosal formulation and a external application.

22. A pharmaceutical composition for prevention or treatment of thrombocytopenia or thrombocytopenia-associated diseases, comprising the TPO fragment variant of any one of claims 1 to 13.

23. A method for treating thrombocytopenia or thrombocytopenia-associated diseases, comprising administering the TPO fragment variant having the modified amino acid sequence of any one of claims 1 to 13 in a therapeutically effective amount to a patient in need thereof.

24. A method of using the TPO fragment variant having the modified amino acid sequence of any one of claims 1 to 13 in treatment of thrombocytopenia or thrombocytopenia-associated diseases.

25. Use of the TPO fragment variant having the modified amino acid sequence of any one of claims 1 to 13 in manufacture of therapeutics for thrombocytopenia or thrombocytopenia-associated diseases.
